(19) **Europäisches Patentamt**

**European Patent Office**

**Office européen des brevets**

(11) **EP 1 722 344 A1**

(12) **EUROPEAN PATENT APPLICATION**

(43) Date of publication:
**15.11.2006 Bulletin 2006/46**

(51) Int Cl.:
*G09B 23/28* (2006.01)   *G06F 17/50* (2006.01)
*G06F 19/00* (2006.01)

(21) Application number: **06009775.5**

(22) Date of filing: **11.05.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **11.05.2005 JP 2005138868**

(71) Applicant: **SYSMEX CORPORATION**
**Kobe-shi,**
**Hyogo 651-0073 (JP)**

(72) Inventors:
• **Kouchi, Yasuhiro**
**Sysmex Corporation**
**Kobe-shi**
**Hyogo 651-0073 (JP)**

• **Saitou, Takeo**
**Sysmex Corporation**
**Kobe-shi**
**Hyogo 651-0073 (JP)**
• **Seiko, Masayoshi**
**Sysmex Corporation**
**Kobe-shi**
**Hyogo 651-0073 (JP)**
• **Naitou, Yasuhiro**
**Fujisawa-shi**
**Kanagawa 252-0816 (JP)**

(74) Representative: **HOFFMANN EITLE**
**Patent- und Rechtsanwälte**
**Arabellastrasse 4**
**81925 München (DE)**

(54) **Biological simulation system and computer program product**

(57)     With an object to seek parameters of a biological model corresponding to individual patients, the present invention provides a biological simulation system comprising an internal parameter set generating section which generates internal parameter sets suitable for use with a biological model, and a biological model computing section computing output of a biological model which emulates a biological response of a biological organ based on the generated internal parameter set, wherein the internal parameter set generating section comprises a means for automatically generating a plurality of different internal parameter sets, and a selecting means which determines a degree of similarity between a biological model output calculated applying an automatically generated internal parameter set and an actual biological response corresponding to said output for each of the automatically generated data sets and which selects an appropriate internal parameter set from a plurality of the generated internal parameter sets.

EP 1 722 344 A1

**Description**

**TECHNICAL FIELD**

The present invention relates to a biological simulation system, particularly a system and a computer program product for simulating pathological condition of diabetes.

**BACKGROUND**

Biological bodies have been conventionally tried to describe by mathematical models. The minimal model by Bergman can be referred to for this model. Bergman's minimal model was disclosed in "American Journal of Physiology, 1979, Vol. 236-6, p.E-667-77, Bergman et al." and "Journal of Clinical Investigation, 1981, Vol. 68-6, p.1456-67".
In this minimal model, variables are blood glucose level, plasma insulin concentration, and insulin action level i.e. remote insulin of insulin action point of a peripheral tissue. The equations of the minimal model are as follows:

$$dG(t) \ / \ dt = -p_1(G(t) - G_b) - X(t)G(t)$$

$$dX(t) \ / \ dt = -p_2X(t) + p_3(I(t) - I_b)$$

$$dI(t) \ / \ dt = -n(I(t) - I_b) + \gamma (G(t) - h)$$
$$(G(t) > h)$$
$$= -n (I(t) - I_b) + \gamma (G(t) - h)$$
$$(G(t) <= h)$$

where blood glucose level for time "t" is represented by "G (t) " , plasma insulin concentration is "I (t)", and remote insulin is "X(t)", and time difference is on the left sides. Parameters in the equation are:

$p_1$ : insulin-independent glucose metabolism rate
$G_b$ : blood-glucose level
$p_2$ : insulin uptake efficiency
$p_3$ : insulin consumption rate against insulin-dependent glucose metabolism
$I_b$ : insulin concentration value
n : insulin consumption per unit time
$\gamma$ : insulin secretion sensitivity against glucose stimulation,
h : threshold level of blood glucose starting insulin secretion. These values depend on individuals.

If we try to simulate a biological body by applying such model to an individual patient and use the model for diagnosis and the like, we need to appropriately set the above-mentioned parameters constituting the biological model depending on the individual patients.
That means, when we try to reproduce an actual patient body by the biological model, we need accuracy of the above-mentioned parameters and obtain accurate parameters different among individual patients as much as possible.

**SUMMARY**

An object of the present invention is to provide a technical means for obtaining parameters of biological models corresponding to individual patients.
A first invention is a biological simulation system using a biological model comprising an internal parameter set generating section generating internal parameter sets constituting a biological model, and a biological model computing section computing output of a biological model which emulates a biological response of a biological organ based on the generated internal parameter set, wherein said internal parameter set generating section comprises means for automatically generating a plurality of different internal parameter sets, and a selecting means which determines an approximation between a biological model output calculated applying the automatically generated internal parameter set and an actual biological response corresponding to said output and which selects an appropriate internal parameter set from a plurality of the generated internal parameter sets.

A second invention is a biological simulation system using a biological model comprising an internal parameter set generating section generating internal parameter sets constituting a biological model, and a biological model computing section simulating a biological organ based on the generated internal parameter set, wherein said internal parameter set generating section comprises means for automatically generating a plurality of different internal parameter sets, and a selecting means which determines an approximation between a biological organ condition shown by a parameter value included in the generated internal parameter set and a biological organ condition shown by a physiological index obtained from an actual body examination and which selects an appropriate internal parameter set from a plurality of generated internal parameter sets.

A third invention is a biological simulation system using a biological model comprising an internal parameter set generating section generating internal parameter sets constituting a biological model, and a biological model computing section simulating a biological organ based on the generated internal parameter set, wherein said internal parameter set generating section comprises means for automatically generating a plurality of different internal parameter sets, and an obtaining means for obtaining internal parameter set showing a biological organ condition approximate to the biological organ condition shown by a biological index which is obtained from an actual body examination, based on the generated internal parameter set.

A fourth invention is a biological simulation system using a biological model comprising an internal parameter set generating section generating internal parameter sets constituting a biological model, and a biological model computing section computing output of the biological model which emulates a biological response of a biological organ based on the generated internal parameter set, wherein said internal parameter set generating section comprises means for automatically generating a plurality of different internal parameter sets, and a selecting means which determines an approximation between the biological model output calculated applying the internal parameter set automatically generated and an actual biological response corresponding to said output and which selects an appropriate internal parameter set from a plurality of the generated internal parameter sets, and an obtaining means for obtaining an appropriate internal parameter set showing a biological organ condition approximate to the biological organ condition shown by the biological index which is obtained from an actual body examination, based on a plurality of the internal parameter sets selected by said selecting means.

Further, with regard to an invention related to a computer program product, a computer is executed to perform the biological simulation as the biological simulation system.

## DESCRIPTION OF DRAWINGS

FIG. 1 is a block diagram showing a hardware construction of a system of the present invention.
FIG. 2 is a block diagram showing overall construction of a biological model.
FIG. 3 is a block diagram showing a construction of pancreas model of the biological model.
FIG. 4 is a block diagram showing a construction of a hepatic metabolism model of the biological model.
FIG. 5 is a block diagram showing a construction of insulin kinetics model.
FIG. 6 is a block diagram showing a construction of a peripheral tissue model.
FIG. 7 is a flowchart showing a parameter generation process.
FIG. 8 is a OGTT time-series datum, (a) is a measured blood-glucose level, and (b) is a measured blood-insulin concentration.
FIG. 9 is a flowchart showing a genetic algorithm.
FIG. 10 is a measured blood glucose level as a reference.
FIG. 11 is output of a biological model applied with a generated parameter set (individual) PS#01.
FIG. 12 is a graph showing error based on a reference of generated individual PS#01.
FIG. 13 is a diagram illustrating crossover of genetic algorithm.
FIG. 14 is a diagram showing a set of a hundred generated parameters.
FIG. 15 is a diagram showing the result of process of standardizing the parameter set of FIG. 14.
FIG. 16 is a dendrogram showing a result of cluster analysis of parameter set.
FIG. 17 is a diagram showing a biological function profile.
FIG. 18 is a first-half part of a flow chart of algorithm calculating reference score based on which a body function profile is selected.
FIG. 19 is a second-half part a flow chart of algorithm calculating reference score based on which a body function profile is selected.

## DETAILED DESCRIPTION OF THE PREFERRED EMBODIMENT

Embodiments of the present invention is described hereinafter with reference to drawings.

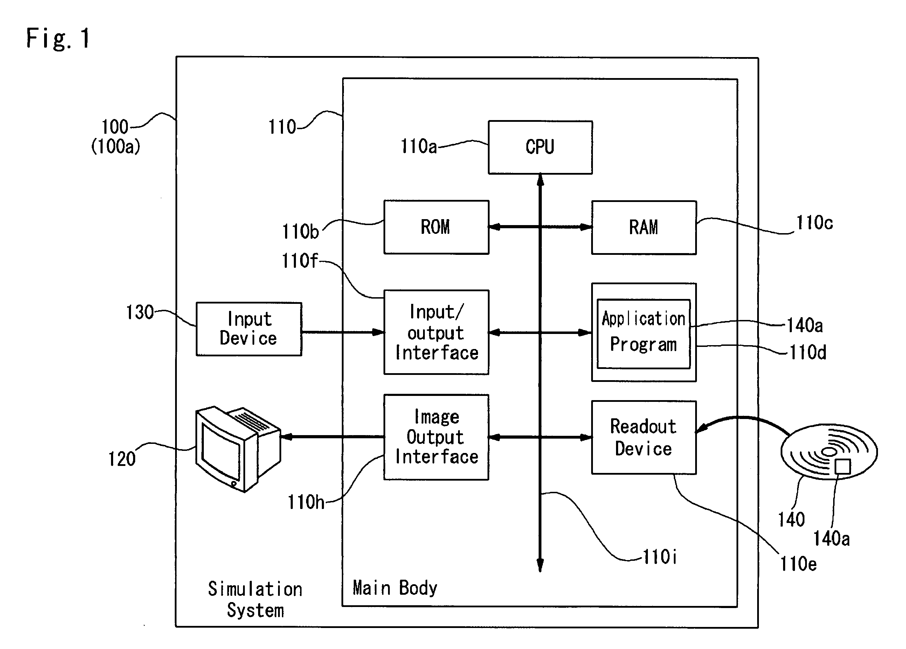

FIG. 1 is a block diagram showing a hardware construction of a biological simulation system (also referred to as "system" hereinafter) related to a first embodiment of the present invention. A system 100 related to the present embodiment is composed of a computer 100a primarily comprising a main body 110, a display 120, and an input device 130. The main body 110 comprises a CPU 110a, a ROM 110b, a RAM 110c, a hard disk 110d, a readout device 110e, an input/output interface 110f, and an image output interface 110h. The CPU 110a, the ROM 110b, the RAM 110c, the hard disk 110d, the readout device 110e, the input/output interface 110f, and the image output interface 110h are data-communicably connected by a bus 110i.

The CPU 110a is capable of executing a computer program recorded in the ROM 110b and a computer program loaded in the RAM 110c. And the CPU 110a executes an application program 140a as described later to realize each function block as described later, thereby the computer 100a functions as the system 100.

The ROM 110b comprises mask ROM, PROM, EPROM, EEPROM, etc. and is recoded with computer programs executed by the CPU 110a and data used for the programs.

The RAM 110c comprises SRAM, DRAM, etc. The RAM 110c is used to read out computer programs recorded in the ROM 110b and the hard disk 110d. And the RAM 110c is used as a work area of the CPU 110a when these computer programs are executed.

The hard disk 110d is installed with an operating system, an application program, etc., various computer programs to be executed by the CPU 110a, and data used for executing the computer programs. An application program 140a described later is also installed in this hard disk 110d.

The readout device 110e which comprises a flexible disk drive, a CD-ROM drive or DVD-ROM drive is capable of reading out a computer program or data recorded in a portable recording media 140. And the portable recording media 140 stores the application program 140a to function as a system of the present invention. The computer 100a reads out the application program 140a related to the present invention from the portable recording media 140 and is capable of installing the application program 140a in the hard disk 110d.

In addition to that said application program 140a is provided by the portable recording media 140, said application program 140a may be provided through an electric communication line (wired or wireless) from outside devices which are communicably connected to the computer 100a via said electric communication line. For example, said application program 140a is stored in a hard disk in an internet server computer to which the computer 100a accesses and said application program 140a may be downloaded and installed in the hard disk 110d.

The hard disk 110d is installed with an operating system which provides a graphical user interface environment, e.g. Windows (trademark) manufactured by US Microsoft corp. In the explanation hereinafter, the application program 140a related to this embodiment shall operate on said operating system.

The input/output interface 110f comprises a serial interface, e.g. USB, IEEE1394, RS-232C, etc.; a parallel interface, e.g. SCSI, IDE, IEEE1284, etc.; and an analog interface e.g. D/A converter, A/D converter, etc. The input/output interface 110f is connected to the input device 130 comprising a keyboard and a mouse and users can input data into the computer 100a using the input data device 130.

The image output interface 110h is connected to the display 120 comprising LCD, CRT or the like so that picture signals corresponding to image data provided from the CPU 110a are output to the display 120. The display 120 displays a picture (screen) based on input picture signals.

[Overall construction of a biological model]

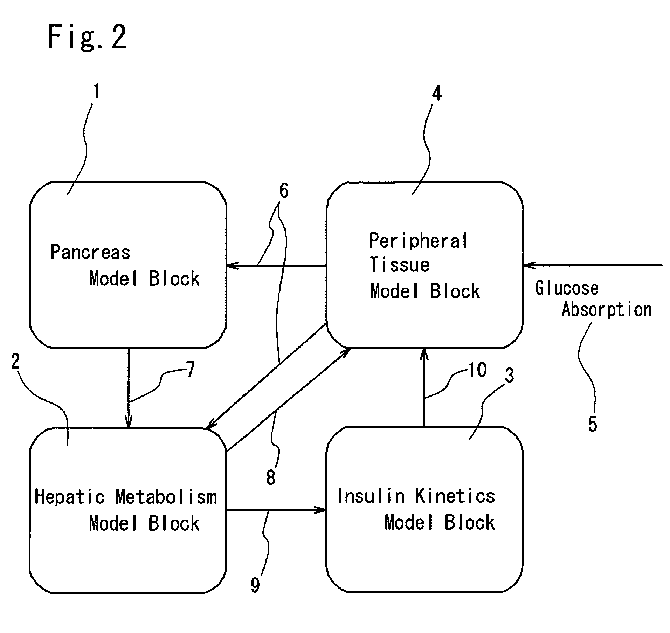

FIG. 2 is a block diagram showing an overall construction of one example of a biological model (biological mathematical model) used in the system of the present invention. As in FIG. 2, an computing unit used in this biological system comprises a pancreas model block (pancreas model block computing unit) 1, a hepatic metabolism model block (hepatic metabolism model block computing unit) 2, an insulin kinetics model block (insulin kinetics model block computing unit) 3, and a peripheral tissue model block (peripheral tissue model block computing unit) 4, each of which simulates biological organs and has input provided outside the biological model or from other blocks and output to other blocks.

That means, the pancreas model block 1 computes in emulation of a pancreas function. A blood glucose level 6 is set as input and an insulin secretion rate 7 is set as output to other blocks.

The hepatic metabolism model block 2 computes in emulation of a hepatic function. A blood glucose level 6 and an insulin secretion rate 7 are set as input and net glucose release 8 and posthepatic insulin 9 are set as output to other blocks.

The insulin kinetics model block 3 computes in emulation of insulin kinetics. Posthepatic insulin 9 is set as input and peripheral tissue insulin concentration 10 is set as output to other blocks.

The peripheral tissue model block 4 computes in emulation of peripheral tissue function. A net glucose release 8, external glucose absorption 5 from outside, and insulin concentration 10 in the peripheral tissue are set as input and a blood glucose level 6 is set as output to other blocks.

Said glucose absorption is a data provided from outside and is performed by user inputting inspection data and the like using, for example, the input device 130. Further, the function blocks 1 to 4 are each realized by the CPU 110a executing the computer program 140a.

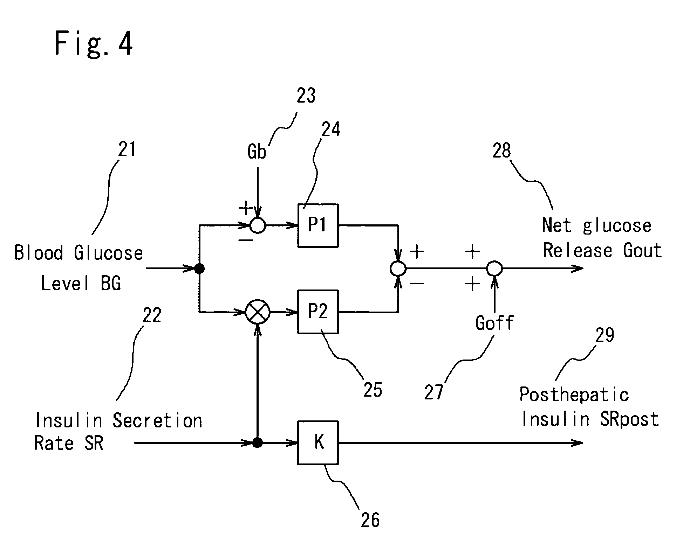

Except for the output to be given to other blocks, there exist values which are calculated in the block 3 but not given to other blocks as they are like the blood-insulin concentration $I_1$ (FIG. 4) in the insulin kinetics model block 3. Such values are also regarded as values obtained from the biological model in terms of the biological model as a whole and the values therefore may be included in the output of the biological model.

[Pancreas Model Block]

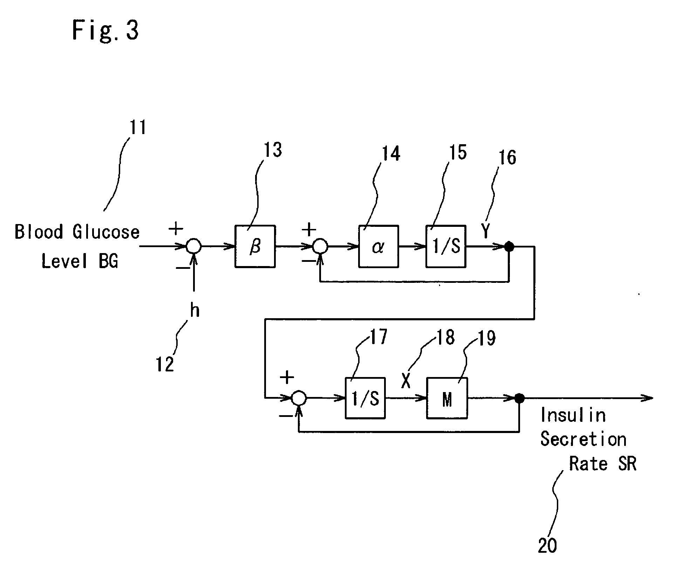

Relationship between input and output of the pancreas model block 1 will be expressed using the following differential equation 1 . A block diagram as in FIG. 3 equivalent to the differential equation 1 is also used.
Differential equation 1:

$$dY/dt = -\alpha\{Y(t) - \beta(BG(t) - h)\} \qquad (BG(t) > h)$$
$$= -\alpha Y(t) \qquad (BG(t) <= h)$$

$$dX/dt = -M \cdot X(t) + Y(t)$$

$$SR(t) = M \cdot X(t)$$

Variables:

BG(t): blood glucose level
X(t) : total amount of insulin capable of secretion from pancreas
Y (t) : supply rate of insulin newly supplied for glucose stimulation
SR(t): pancreas insulin secretion rate

Parameters:

h: threshold of glucose concentration capable of stimulating insulin supply
$\alpha$: following performance to glucose stimulation
$\beta$: sensitivity to glucose stimulation
M: secretion rate per unit concentration

where a blood glucose level 6 which is input to the pancreas model block in FIG. 2 corresponds to BG(t). The insulin secretion rate 7 in FIG. 2 which is output of the pancreas model block corresponds to SR(t).

In FIG. 3, numeral 11 is blood glucose level: BG, 12 is glucose concentration threshold stimulating insulin supply: h, 13 is glucose stimulation sensitivity: $\beta$, 14 is glucose stimulation following capability: $\alpha$, 15 is integral element, 16 is supply rate of insulin newly supplied for glucose stimulation: Y, 17 is integral element, 18 is total amount of insulin capable of secretion from pancreas: X, 19 is secretion rate per unit concentration: M, 20 is pancreas insulin secretion rate: SR.

[Hepatic Metabolism Model Block]

Relationship between input and output of the hepatic metabolism model block 2 will be described using the following differential equation 2. A block diagram as in FIG. 4 equivalent to the differential equation 2 is also used.
Differential equation 2:

$$RGout(t) = P1(Gb - BG(t)) - P2 \cdot SR(t) \cdot BG(t) + Goff$$
$$(BG(t) < Gb)$$
$$= - P2 \cdot SR(t) \cdot BG(t) + Goff \quad (BG(t) >= Gb)$$

$$SRpost(t) = K \cdot SR(t)$$

Variables:

BG(t): blood glucose level
SR(t): pancreas insulin secretion rate
RGout(t): net glucose from liver
SRpost(t): posthepatic insulin

Parameters:

Gb: glucose concentration basic value
P1 : glucose production rate to glucose stimulation lower than Gb
P2: hepatic glucose uptake rate per unit insulin and unit glucose
K: insulin uptake rate in liver
Goff: glucose release rate to basal metabolism

where the blood glucose level 6 which is input to the hepatic metabolism model block in FIG. 2 corresponds to BG(t) and the insulin secretion rate 7 corresponds to SR(t) . The net glucose release 8 which is output of the hepatic metabolism model block in FIG. 2 corresponds to RGout(t) and the posthepatic insulin 9 corresponds to SRpost(t).

In FIG. 4, numeral 21 is blood glucose level: BG, 22 is pancreas insulin secretion rate: SR, 23 is glucose concentration basic value: Gb, 24 is glucose production rate to glucose stimulation lower than Gb: P1, 25 is liver glucose uptake rate per unit insulin and per unit glucose: P2, 26 is liver insulin uptake rate: K, 27 is glucose release rate to basal metabolism: Goff, 28 is net glucose from liver: RGout, 29 is posthepatic insulin: SRpost.

[Insulin Kinetics Model Block]

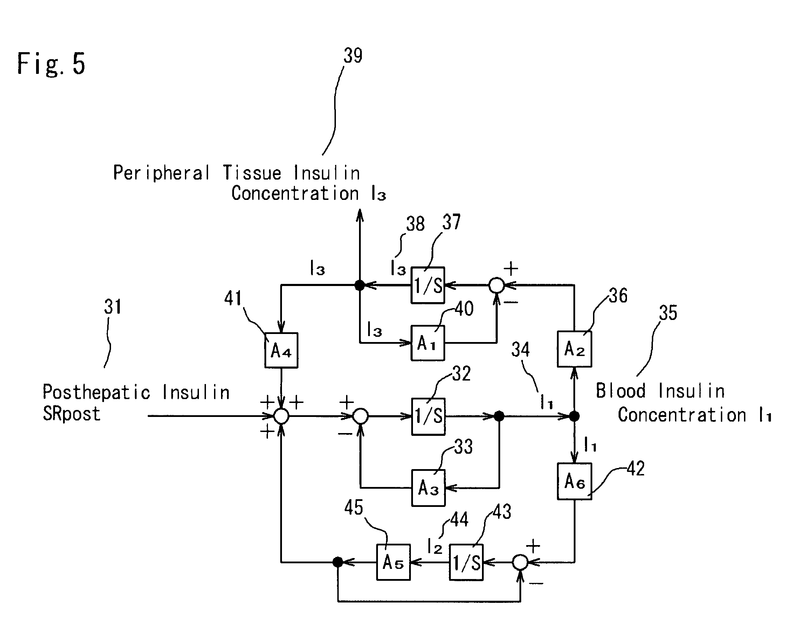

Relationship between input and output of the insulin kinetics model block 3 will be described using the following differential equation 3. A block diagram as in FIG. 5 equivalent to the differential equation 3 is also used.
Differential equation 3:

$$dI_1(t) / dt = - A_3 I_1(t) + A_5 I_2(t) + A_4 I_3(t) + SRpost(t)$$

$$dI_2(t) / dt = A_6 I_1(t) - A_5 I_2(t)$$

$$dI_3(t) / dt = A_2 I_1(t) - A_1 I_3(t)$$

Variables:

SRpost(t): posthepatic insulin
$I_1(t)$: blood insulin concentration
$I_2(t)$: insulin concentration in insulin-independent tissue
$I_3(t)$: insulin concentration in peripheral tissue

Parameters:

A1: disappearance rate in peripheral tissue
A2: insulin distribution rate in peripheral tissue
A3: insulin uptake rate in liver
A4: post peripheral tissue insulin flow out rate
A5: insulin disappearance rate in insulin-independent tissue
A6: insulin distribution rate to insulin-independent tissue

where the posthepatic insulin 9 which is input to the insulin kinetics model block in FIG. 2 corresponds to SRpost (t). The peripheral tissue insulin concentration 10 which is output to the insulin kinetics model block in FIG. 2 corresponds to $I_3(t)$.

In FIG. 5, 31 is posthepatic insulin: SRpost, 32 is integral element, 33 is insulin uptake rate in liver : A3, 34 and 35 are blood insulin concentration : $I_1$, 36 is insulin distribution rate to peripheral tissue: A2, 37 is integral element, 38 and 39 are insulin concentration in peripheral tissue: $I_3$, 40 is insulin disappearance rate in peripheral tissue: A1, 41 is post peripheral tissue insulin discharge rate: A4, 42 is insulin distribution rate to insulin-independent tissue: A6, 43 is integral element, 44 is insulin concentration in insulin-independent tissue: $I_2$, 45 is insulin disappearance rate in insulin-independent tissue: A5.

[Peripheral tissue Model Block]

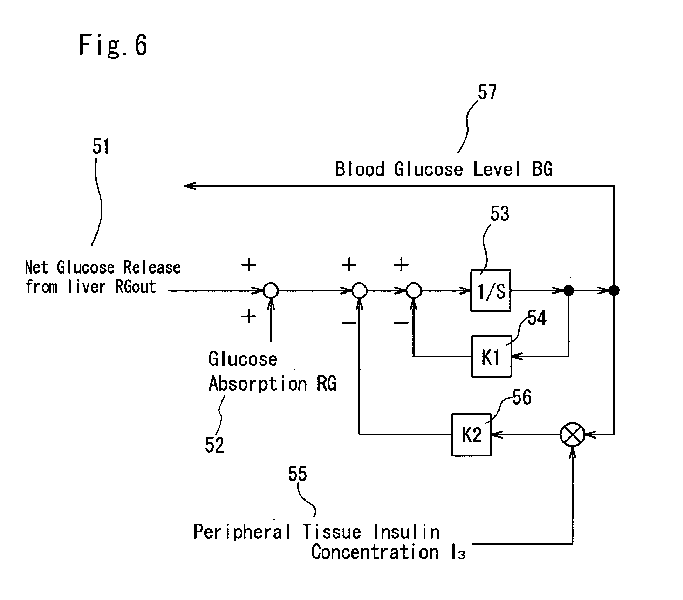

Relationship between input and output of the peripheral tissue model block 4 will be described using the following differential equation 4. A block diagram as in FIG. 6 equivalent to the differential equation 4 is also used.
Differential equation 4:

$$\text{dBG(t) / dt = -K1.BG(t) - K2.I}_3\text{(t)} \cdot \text{BG(t) + RG(t) + RGout(t)}$$

Variables:

BG(t): blood glucose level
RG(t): glucose adsorption from digestive tract
RGout(t): net glucose from liver
$I_3(t)$: insulin concentration in peripheral tissue

Parameters:

K1: insulin-independent glucose consumption rate in peripheral tissue
K2: insulin-dependent glucose consumption rate in peripheral tissue

where the peripheral tissue insulin concentration 10 which is input to the peripheral tissue model block in FIG. 2 corresponds to $I_3(t)$, the net glucose 8 from liver corresponds to RGout(t) and the glucose absorption 5 from digestive tact 5 corresponds to RG(t). The blood glucose level 6 which is output of the peripheral tissue model block corresponds to BG(t).

In FIG. 6, numeral 51 is net glucose from liver: RGout, 52 is glucose adsorption from digestive tract: RG, 53 is integral element, 54 is insulin-independent glucose consumption rate in peripheral tissue: K1, 55 is insulin concentration in peripheral tissue: $I_3$, 56 is insulin-dependent glucose consumption rate in peripheral tissue: K2, 57 is blood glucose level: BG.

Each block outputs time-series change of each output item based on the above-mentioned differential equation. Further, as in FIG. 2, input/output between blocks constituting the present system is connected to each other and output of a certain block gives input of the other block, so that output of each block changes according to the time-series change of the block output. Therefore, for example, when glucose absorption from digestive tract: RG is input in the biological model from the input device 130, time-series change of values of blood glucose level: BG (t) and blood insulin concentration: $I_1(t)$ are calculated and simulated based on the mathematical formulas.

Thus, the blood glucose level and the insulin concentration which have been sequentially calculated in such way can be displayed in the display 120. Thereby users can easily confirm results of the biological organ simulation as mentioned above. Further, it is possible to employ the present system as a subsystem for simulating biological functions in a medical system such as a diabetes diagnosis supporting system. In this case, the time-series change of calculated blood glucose level and insulin concentration is passed to other components of medical systems, by which, for example, diabetes diagnosis supporting information is provided. It is possible to obtain reliable medical information based on the blood glucose level and insulin concentration calculated by the present system.

With regard to calculation of the differential equations of the present system, e.g. E-cell (software disclosed by Keiou University) and MATLAB (manufactured by the MathWorks Inc.) may be employed. Or other calculation system may be employed.

[Parameter Set Generating Section]

The present simulation system has a parameter set generation function (parameter set generating section) which obtains a internal parameter set of the biological model (also simply referred to as "parameter set" hereinafter). The parameter set generated by said function is provided to said biological model and a biological model computing unit simulates functions of the biological organs.

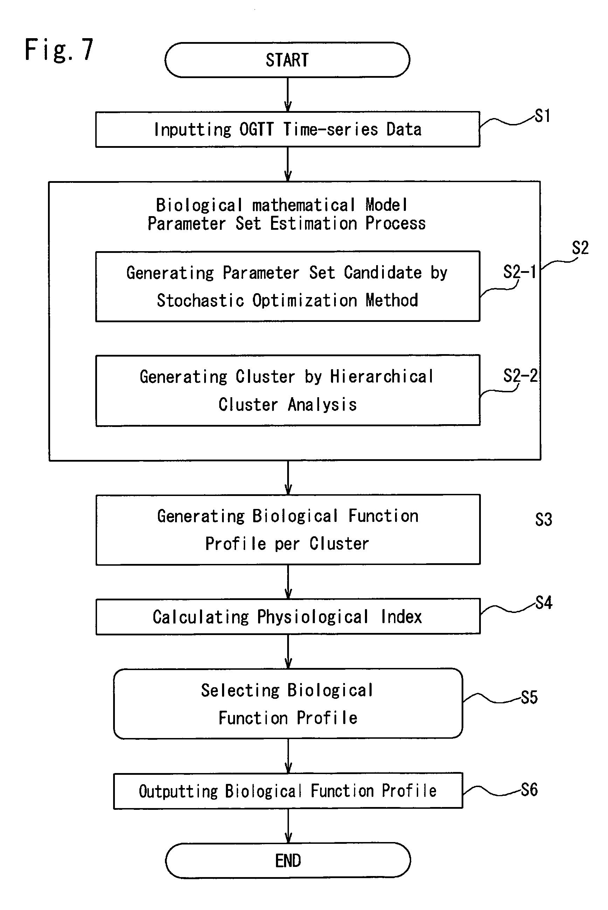

FIG. 7 is a diagram showing procedures in which the parameter set generating section of the present system obtains a parameter set of the biological model. As shown in the diagram, the procedure of obtaining parameters comprises a step (S1) of inputting OGTT (oral Glucose Tolerance Test), and time-series data (blood glucose level change data, insulin concentration change data), a step (S2) of estimating a candidate of a biological mathematical model parameter set, a step (S3) of generating a biological function profile, a step (S4) of inputting actual diagnosis data, a step (S5) of calculating a biological index, and a step (S6) of selecting appropriate biological profile.

[Step 1: inputting OGTT time-series data]

OGTT time-series data are a result of OGTT (given amount of glucose solution is orally loaded to measure the time-series of blood glucose level and insulin concentration) from the actual examination of patients simulated by a biological model. The present system receives input as an actual biological response. The input OGTT time-series data are used as a basis and others for estimating a candidate of internal parameter set.

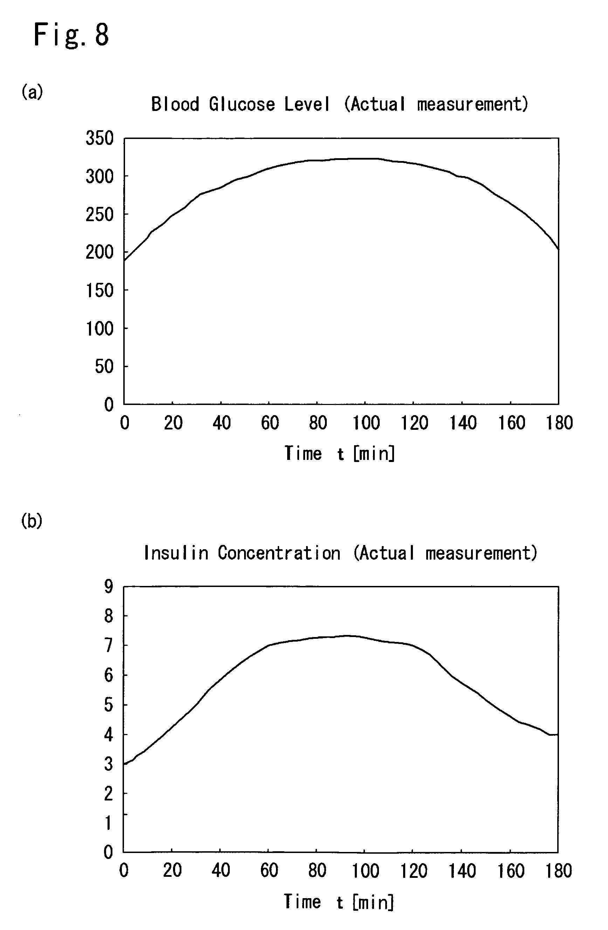

FIG. 8 shows blood glucose level change data as OGTT time-series data (FIG. 8 (a)) and blood insulin concentration change data (FIG. 8 (b)).

In FIG. 8 (a), the blood glucose level change data is measured data corresponding to time-series change of blood glucose level BG (t), one of output items in the biological model shown in FIGs. 2 to 6.

In FIG. 8 (b), the blood insulin concentration change data is measured data corresponding to time-series change of blood insulin concentration $I_1$ (t) , one of output items in the biological model shown in FIGs. 2 to 6.

For inputting the OGTT time-series data to the present system, an input device 130 such as a keyboard and a mouse may be used. Or external memory device such as a database previously registered with OGTT time-series data.

[Parameter to be estimated]

In the biological model shown in FIGs. 2 to 6, as parameters, required are [h, $\alpha$, $\beta$, M] for the pancreas model, [Gb, P1, P2, Goff, K] for the hepatic metabolism model, [A1, A2, A3, A4, A5, A6] for the insulin kinetics model, [K1, K2] for the peripheral tissue model. For calculating differential equations, as an initial value of variable required are [X (0), Y(0), BG(0)] for the pancreas model, [$I_1$(0), $I_2$(0), $I_3$(0)] for insulin kinetics model. The initial value of variable below shall be included in parameters to be estimated unless otherwise specified.

In said biological model, there exist 23 parameters including initial value of variable. In the process of estimating candidate of parameter set, a parameter set PS consisting of 19 parameters out of 23 parameters is estimated.

Particularly, among 23 parameters, a blood glucose level initial value BG(0) and a blood insulin concentration initial value $I_1$(0) can be determined by an initial value (value at t=0) of observed value of FIGs. 8 (a), 8 (b), thereby estimation is not necessary. Further, in the present embodiment, an insulin concentration initial value of insulin independent tissue $I_2$(0) and an insulin concentration initial value of peripheral tissue $I_3$(0) are fixed at 0, thereby estimation is not necessary either. Thus, 19 remaining parameters are subject to estimation (ref. to a later-mentioned table 1). Hereinafter, 19 parameters shall be referred to as "Px" (x : 01 to 19) for convenience of explanation. Relationship between Px and each parameter marks is shown in the table 1 mentioned later.

[Step S2: estimating parameter set candidates of a biological mathematical model]

In a step of estimating parameter set candidate, Parameter set PS candidates of a biological mathematical model where time-series data shown in FIGs. 8(a),(b) can be reproduced in a given error range is obtained by a stochastic optimization method (step S2-1). The parameter set PS candidates which have been obtained by a stochastic optimization method are narrowed down by Hierarchical cluster analysis (Step S2-2).

Genetic algorithm (is also simply referred to as "GA" hereinafter) is used for the stochastic optimization method here. The following is a procedure of estimating a plurality of parameter set candidates based on GA.

Here, one parameter set PS candidate is obtained per one GA mentioned below and GA is repeated 100 times to generate 100 sets of biological model parameter set candidates (PS#01 to PS#100). That means, it is possible to automatically generate a plurality of different internal parameter sets by repeating multiple times of genetic algorithm for generating internal parameter sets. The number of parameter set PS candidates obtained by stochastic optimization at a time may be one or more.

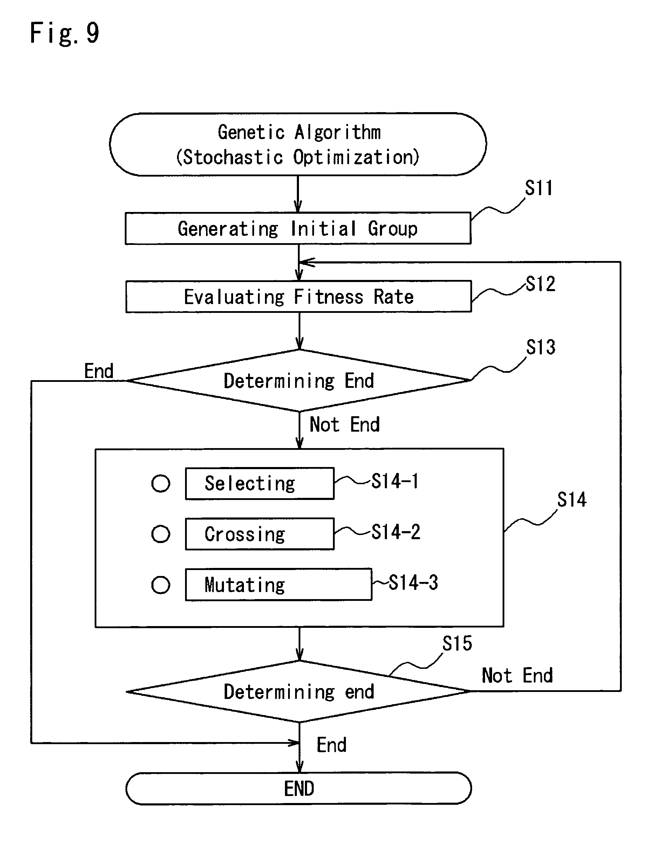

The procedure of generating the parameter set candidates by GA comprises, as shown in FIG. 9, a step of generating initial group of parameter set (Step S11), a step of evaluating fitness (Step S12), a step of selecting, crossing over, and mutating (Step S14), and a step of determining end (Step S13, S15) . The steps of generating initial group (Step S11) and selecting, crossing over, and mutating (Step S14) correspond to the step of automatically generating internal parameter set (candidate) in the present invention.

The algorithm in FIG. 9 will be described in detail herein after.

[Step S11: generating initial group]

This system has search-range information for each of biological model parameters P01 to P19 as shown in the following table 1. The system has functions to generate random numbers per parameter within a range of maximum value and minimum value of the table 1, thereby automatically generating parameter set PS within the predetermined search range. The parameter set PS obtained in this way may be referred to as "individual".

[Table 1]

| Each Parameter Search Range | | | |
|---|---|---|---|
| Parameter | | Minimum | Maximum |
| P01 | h | 24 | 100 |
| P02 | β | 2. 5 | 11 |
| P03 | α | 35 | 142 |
| P04 | Y (0) | 83 | 335 |
| P05 | M | 0.005 | 0.02 |
| P06 | X (0) | 1150 | 4600 |
| P07 | Gb | 40 | 160 |
| P08 | P1 | 0.0005 | 0.0024 |
| P09 | P2 | 2.00E-06 | 8.50E-06 |
| P10 | Goff | 1.9 | 7.6 |
| P11 | K | 0.048 | 0.2 |
| P12 | A3 | 0.2 | 0.88 |
| P13 | A5 | 0.01 | 0.045 |
| P14 | A6 | 0.095 | 0.4 |
| P15 | A 1 | 0.03 | 0.15 |
| P16 | A2 | 0.025 | 0.12 |
| P17 | A4 | 0.02 | 0.085 |
| P18 | K1 | 0.015 | 0.07 |
| P19 | K2 | 0.0002 | 0.00095 |

An initial group consisting of multiple parameter sets PS is generated by repeating multiple times of the step of generating random numbers every 19 parameters within a search range.

The following table 2 shows examples of 10 parameter sets PS#01 to PS#10 which are generated as an initial group.
[Table 2]

| | Initial Group | | | | | | | | | |
|---|---|---|---|---|---|---|---|---|---|---|
| Parameter | PS#01 | PS#02 | PS#03 | PS#04 | PS#05 | PS#06 | PS#07 | PS#08 | PS#09 | PS#10 |
| P01 | 77.853 | 90.646 | 86.601 | 82.018 | 94.53 | 83.308 | 84.051 | 96.727 | 73.679 | 92.607 |
| P02 | 10.254 | 8.5714 | 9.3763 | 9.0354 | 9.5593 | 6.3751 | 8.0237 | 6.6845 | 9.1471 | 8.5071 |
| P03 | 76.848 | 77.44 | 81.342 | 103.76 | 56.412 | 114.08 | 93.708 | 70.688 | 84.96 | 56.707 |
| P04 | 186.15 | 300.74 | 180.43 | 333.56 | 274.36 | 250.62 | 94.813 | 278.29 | 202.31 | 176.09 |
| P05 | 0.019609 | 0.015203 | 0.014726 | 0.019422 | 0.018268 | 0.018808 | 0.01509 | 0.015303 | 0.02 | 0.019746 |
| P06 | 2997.4 | 3008.6 | 3815.3 | 2493.3 | 4403 | 2195.4 | 3708 | 3418.9 | 3101.4 | 4414.6 |
| P07 | 76.823 | 109.99 | 77.006 | 76.125 | 97.159 | 153.65 | 96.861 | 108.84 | 140.18 | 124.2 |
| P08 | 0.000657 | 0.002311 | 0.001592 | 0. 00173 | 0.001012 | 0.000749 | 0.000926 | 0.002029 | 0.001735 | 0.002074 |
| P09 | 3. 97E-06 | 6. 09E-06 | 6. 86E-06 | 6. 78E-06 | 3. 88E-06 | 5. 01E-06 | 3. 57E-06 | 4. 24E-06 | 2. 49E-06 | 4.99E-06 |
| P10 | 5.1346 | 7.2958 | 7.2995 | 5.0623 | 7.0599 | 3.6222 | 7.2376 | 4.5128 | 6.7474 | 5.4203 |
| P11 | 0.12128 | 0.1855 | 0.1777 | 0.14734 | 0.18251 | 0.1887 | 0.18836 | 0.16826 | 0.10262 | 0.171 |
| P12 | 0.72042 | 0.52806 | 0.7295 | 0.66788 | 0.556 | 0.41407 | 0.61329 | 0.37957 | 0.68823 | 0.56269 |
| P13 | 0.023928 | 0.038092 | 0.04128 | 0.021914 | 0.033111 | 0.034394 | 0.035747 | 0.035786 | 0.037128 | 0.035346 |
| P14 | 0.15371 | 0.19032 | 0.25266 | 0.14058 | 0.14531 | 0.11857 | 0.20006 | 0.13184 | 0.14713 | 0.2135 |
| P15 | 0.038029 | 0.057366 | 0.058979 | 0.038294 | 0.037683 | 0.051213 | 0.049506 | 0.061049 | 0.035388 | 0.046363 |
| P16 | 0.062839 | 0.069199 | 0.075943 | 0.066933 | 0.091455 | 0.045022 | 0.084542 | 0.046748 | 0.08376 | 0.056693 |
| P17 | 0.059673 | 0.030564 | 0.063411 | 0.041225 | 0.037884 | 0.037145 | 0.035402 | 0.028912 | 0.04172 | 0.051222 |
| P18 | 0.020966 | 0.041268 | 0.033837 | 0.024043 | 0.044347 | 0.020292 | 0.042372 | 0.017696 | 0.027471 | 0.027924 |
| P19 | 0.000506 | 0.00068 | 0.000793 | 0.0004 | 0.000269 | 0.000567 | 0.00049 | 0.000854 | 0.000462 | 0.000539 |

**EP 1 722 344 A1**

[Step 12: evaluating fitness]

This system performs fitness evaluation on generated individuals to select and draw some individual PS from individuals PS of the (initial) group.

In the fitness evaluation, observed time-series data (FIGs. 8 (a), 8 (b)) which have been input in the step 1 are used as a reference. The actually measured data (biological body response) used as a reference are the data which this system desires to reproduce as output of the biological model. If the same response with the reference is obtained even in the biological model which is applied with the generated parameter set, it is considered that the individual's fitness for the actually measured value is high.

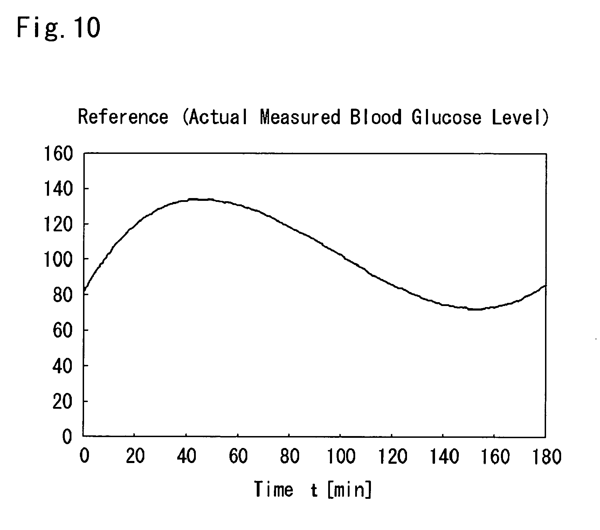

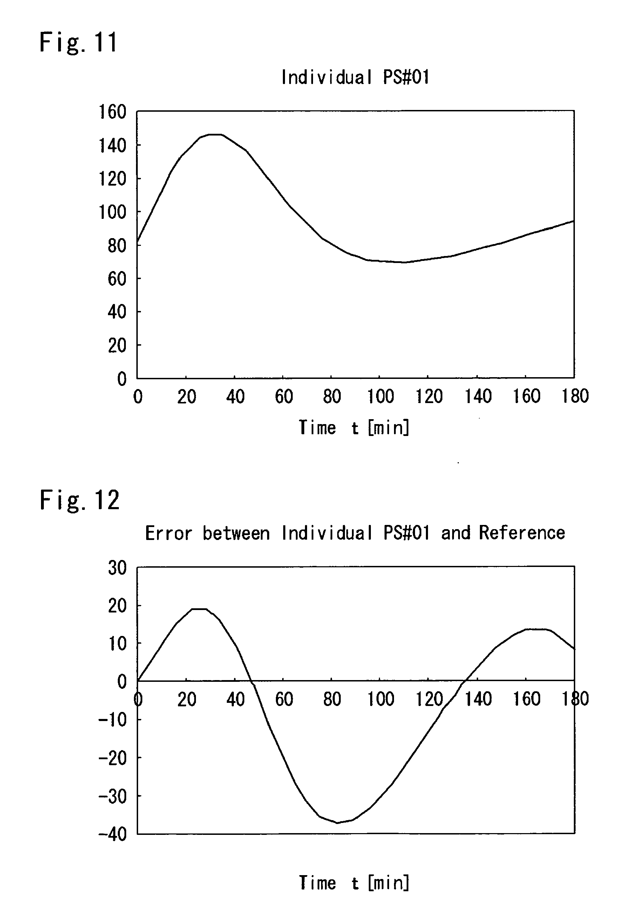

For example, time-series data ref(t) of blood glucose level desired to reproduce in the biological model, is as shown in FIG. 10 and a computed result yl(t) of the blood glucose level in the biological model applied with individual PS#01 parameter is as shown in FIG. 11. In this case, complementary error function e(t) and error value are obtained by the following formulas (1) and (2).

[Formula 1]

$$e(t) = y1(t) - ref(t) \qquad \cdots\cdots\cdots(1)$$

[Formula 2]

$$error = \int_0^{180} \sqrt{[e(t)]^2}\, dt \qquad \cdots\cdots\cdots(2)$$

Here, fitness value f is set in formula (3). If the fitness value f is nearest to 0, the fitness rate shall be highest.

$$f = \overline{error} \cdots \quad (3)$$

[Table 3]

| Individual Fitness Value | |
|---|---|
| Individual Number | Fitness Value |
| PS#01 | 2997.0 |
| PS#02 | 3397.2 |
| PS#03 | 3791.0 |
| PS#04 | 2580.0 |
| PS#05 | 2815.4 |
| PS#06 | 2771.5 |
| PS#07 | 3140. 1 |
| PS#08 | 2812. 7 |
| PS#09 | 3454.3 |
| PS#10 | 2994.8 |

Table 3 shows a result of this system seeking a fitness value f with regard to individual PS#01 to PS#10.

Although, fitness is evaluated only by actually measured blood glucose level among actual biological responses in the above explanation, actually measured insulin concentration may be used for fitness evaluation.

[Step. S14-1: selecting]

Next, in this system, a selection probability F is obtained by Formula (4) based on the fitness value f of the table 3. (#i is the number of an individual, i : 01 to 10)

[Formula 4]

$$F(\#i) = 1 - \frac{f(\#i)}{\sum_{i=1}^{10} f(\#i)} \quad \cdots\cdots (4)$$

The following table 4 shows selection probability F of each individual of the (initial) group obtained by Formula (4) .

[Table 4]

| Individual Selection Probability | |
|---|---|
| Individual Number | Selection Probability |
| PS#01 | 0.902548 |
| PS#02 | 0.889537 |
| PS#03 | 0.876732 |
| PS#04 | 0.916109 |
| PS#05 | 0.908455 |
| PS#06 | 0.909881 |
| PS#07 | 0. 897896 |
| PS#08 | 0.908541 |
| PS#09 | 0.887679 |
| PS#10 | 0.902622 |

In this system, some individuals having high selection probability (for example, 4 individuals: PS#04, PS#06, PS#08, PS#05) are selected from a (initial) group and designated as "parents". As for a selection reference, not only "parents" with high selection probability but also some "parents" with low selection probability may be included in expectation that the fitness rate will increase in "children" the later generation. In this embodiment, selection is based not on the fitness value f with a wide scope of selection value but on selection probability F(0 to 1) with a restricted scope of values, thereby selection is easy with a flexible selection reference.

[Step S14-2: crossing]

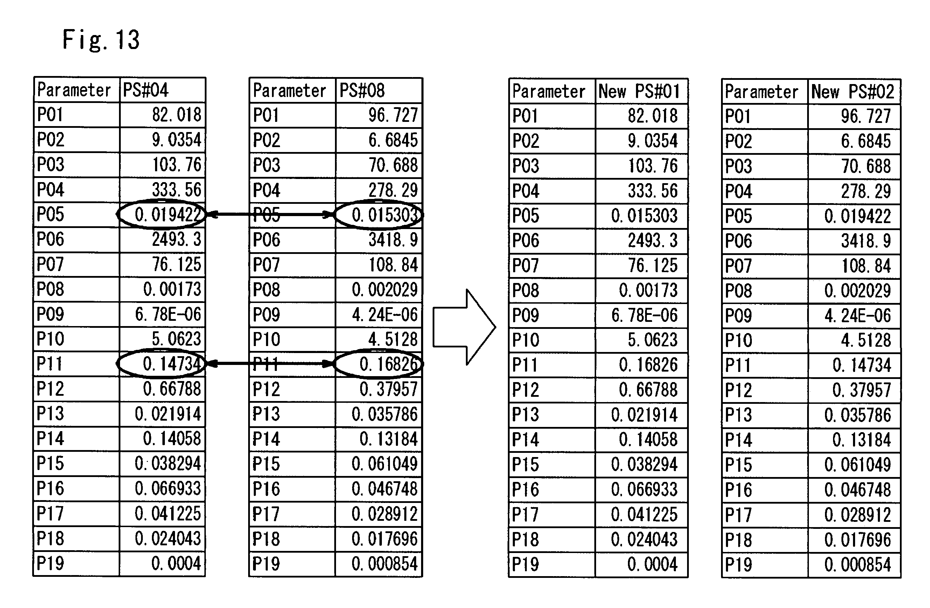

Against the group of individuals (PS#04, PS#06, PS#08, PS#05) selected as "parents" in the above selecting step, new two individuals as "children" are generated by the following procedure in this system. (Ref. to FIG. 13).
First, (1) two individuals are selected at random from the selected group of individuals. Assumed that PS#04 and PS#08 are selected as shown in FIG. 13.
Next, (2) Frequency of crossing with individuals each other is obtained (the number of parameters as subject to be exchanged). The crossing frequency is obtained by the following formula where a crossing probability is expressed by XR (a range of 0 to 1 range):

```
    Crossing frequency = [XR  * (the number of parameters
  held by one individual)][] is a gauss mark ·(e.g.  [3.14]=3)
```

For example, with XR = 0.11, since one individual has nineteen parameters, crossing frequency is [0.11x19] = [2.09] = 2.

XR may be a fixed value or random number.

And then, (3) a crossing point is obtained. The crossing point is obtained by randomly generating integral values from 1 to parameter number (19) at "crossing frequency". For example, with crossing frequency number of 2, random numbers are generated among 1 to 19 two times to obtain 5 and 11.

Finally, (4) new individuals are generated.
Particularly, between 2 individuals (PS#04, PS#08) selected in the step (1), parameters P05 and P11 of the crossing points (5, 11) obtained in the step (3) are exchanged to generate new two individuals (new PS#01, new PS#02).

Repetition of the above steps (1) to (4) generates new individuals "children" (6 individuals in the above example) by as many as the number of individuals which is decreased by selection, and then a new group is generated. The new group is shown in the following Table 5.

EP 1 722 344 A1

[Table 5]

| | New Group | | | | | | | | | |
|---|---|---|---|---|---|---|---|---|---|---|
| Parameter | PS#04 | PS#06 | PS#08 | PS#05 | New PS#01 | New PS#02 | New PS#03 | New PS#04 | New PS#05 | New PS#06 |
| P01 | 82.018 | 83.308 | 96.727 | 94.53 | 82.018 | 96.727 | 83.308 | 82.018 | 94.53 | 96.727 |
| P02 | 9.0354 | 6.3751 | 6.6845 | 9.5593 | 9.0354 | 6.6845 | 6.3751 | 9.0354 | 6.6845 | 9.5593 |
| P03 | 103.76 | 114.08 | 70.688 | 56.412 | 103.76 | 70.688 | 114.08 | 103.76 | 70.688 | 56.412 |
| P04 | 333.56 | 250.62 | 278.29 | 274.36 | 333.56 | 278.29 | 250.62 | 333.56 | 278.29 | 274.36 |
| P05 | 0.019422 | 0.018808 | 0.015303 | 0.018268 | 0.015303 | 0.019422 | 0.019422 | 0.018808 | 0.015303 | 0.018268 |
| P06 | 2493.3 | 2195.4 | 3418.9 | 4403 | 2493.3 | 3418.9 | 2195.4 | 2493.3 | 4403 | 3418.9 |
| P07 | 76.125 | 153.65 | 108.84 | 97.159 | 76.125 | 108.84 | 153.65 | 76.125 | 108.84 | 97.159 |
| P08 | 0.00173 | 0.000749 | 0.002029 | 0.001012 | 0.00173 | 0.002029 | 0.000749 | 0.00173 | 0.002029 | 0.001012 |
| P09 | 6. 78E-06 | 5.01E-06 | 4. 24E-06 | 3.88E-06 | 6. 78E-06 | 4.24E-06 | 6. 78E-06 | 5.01E-06 | 4.24E-06 | 3.88E-06 |
| P10 | 5.0623 | 3.6222 | 4.5128 | 7.0599 | 5.0623 | 4.5128 | 3.6222 | 5.0623 | 4.5128 | 7.0599 |
| P11 | 0.14734 | 0.1887 | 0.16826 | 0.18251 | 0.16826 | 0.14734 | 0.1887 | 0.14734 | 0.16826 | 0.18251 |
| P12 | 0.66788 | 0.41407 | 0.37957 | 0.556 | 0.66788 | 0.37957 | 0.41407 | 0.66788 | 0.37957 | 0.556 |
| P13 | 0.021914 | 0.034394 | 0.035786 | 0.033111 | 0.021914 | 0.035786 | 0.034394 | 0.021914 | 0.035786 | 0.033111 |
| P14 | 0.14058 | 0.11857 | 0.13184 | 0.14531 | 0.14058 | 0.13184 | 0.14058 | 0.11857 | 0.13184 | 0.14531 |
| P15 | 0.038294 | 0.051213 | 0.061049 | 0.037683 | 0.038294 | 0.061049 | 0.051213 | 0.038294 | 0.061049 | 0.037683 |
| P16 | 0.066933 | 0.045022 | 0.046748 | 0.091455 | 0.066933 | 0.046748 | 0.045022 | 0.066933 | 0.046748 | 0.091455 |
| P17 | 0.041225 | 0.037145 | 0.028912 | 0.037884 | 0.041225 | 0.028912 | 0.037145 | 0.041225 | 0.028912 | 0.037884 |
| P18 | 0.024043 | 0.020292 | 0.017696 | 0.044347 | 0.024043 | 0.017696 | 0.020292 | 0.024043 | 0.017696 | 0.044347 |
| P19 | 0.0004 | 0.000567 | 0. 000854 | 0.000269 | 0. 0004 | 0.000854 | 0.000567 | 0.0004 | 0.000269 | 0.000854 |

Against all individuals of the new group shown in Table 5, this system changes parameters P01 to P19 of each individual with mutation probability MR (in the range of 0 to 1) by the following procedures.

For example, in a mutation process conducted on parameter P01 of individual PS#04, random number R is generated in the range of 0 to 1. With R $\leqq$ MR, the random number is generated within the search range of P01 shown in Table 1 and substituted for an original value of P01. The same process is conducted on P02 to P19.

[Step S13, S15: determining end condition]

Steps S 12 to S14 are repeated as shown in Fig. 9. When individual having the highest fitness rate exists in the present group as a result of fitness rate evaluation in the step S12, GA process is terminated and the individual parameter set having the highest fitness rate in the group is regarded as a result of estimation (step S13). A fitness rate of determination condition of end may be e.g. fitness value $\leqq$ 500.

When frequency of repetition steps S12 to S14 (fitness rate evaluation to mutation) exceeds predetermined frequency, the GA procedure is terminated and the individual (parameter set) having the highest fitness rate in the group is the estimation result (step S15) . As a determination condition of end, the repetition frequency may be e.g. 300 times.

Thus, the processes of fitness rate evaluation and end condition determination are performed to determine approximation between output of the biological model which was calculated by applying the automatically generated internal parameter set and an actual biological response which corresponds to the output, thereby an appropriate internal parameter set can be selected from multiple internal parameter sets generated.

[Step S2-2: generating cluster by hierarchical cluster analysis]

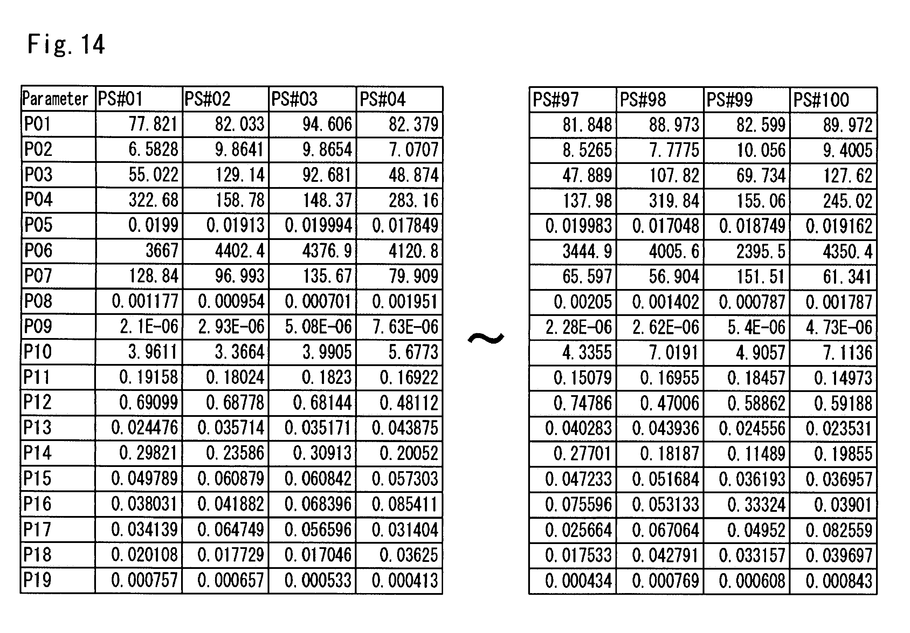

A hundred sets of parameter set PS candidates are obtained by applying the above-mentioned GA a hundred times as shown in FIG. 14. These parameter set PS candidates do not have completely random values which are evenly distributed but they generally have local distribution of parameter values which are approximate to each other. Because it is useless to conduct a process like the below mentioned step S6 on parameter sets having approximate parameter values, "cluster" which groups parameter sets having approximate parameter values is generated here.

For the purpose of the process for generating cluster, this system performs processes of (1) parameter set standardization and (2) hierarchical cluster analysis.

The parameter standardization (normalization) process is pretreatment for the hierarchical analysis cluster. The standardization process is purposed to eliminate influence due to a difference between parameters in unit/numeric value region because each parameter has different unit/numeric values.

Standardization calculation is conducted with CPU 110a by the following way, for example. Parameter P01 in FIG. 14 is standardized using the following formula.

[Formula 5]

$$nP01\,(\#i) = \frac{PS01\,(\#i) - mean\,(PS01)}{SD\,(PS01)} \quad \cdots\cdots\cdots(5)$$

Here,

P01 (#i): parameter P01 of the ith parameter set,
nP01 (#i): standardized P01 (#i),
mean (PS01): mean value of P01 (#1) to P01 (#100)
SD (PS01) : normal deviation value P01 (#1) to P01 (#100)

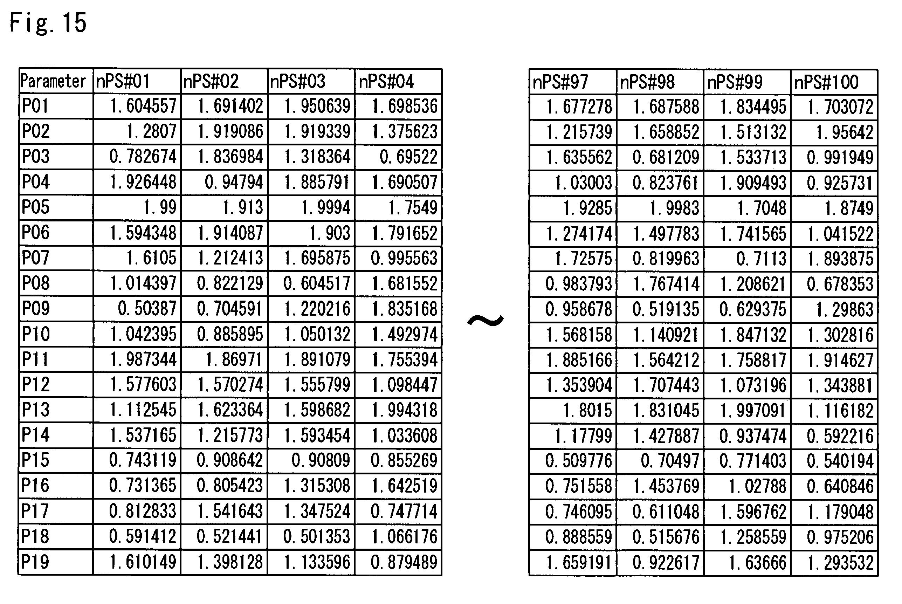

Parameters P02 to P19 except for P01 are also standardized using similar formulas with Formula 5. An example of the result is shown in FIG. 15.

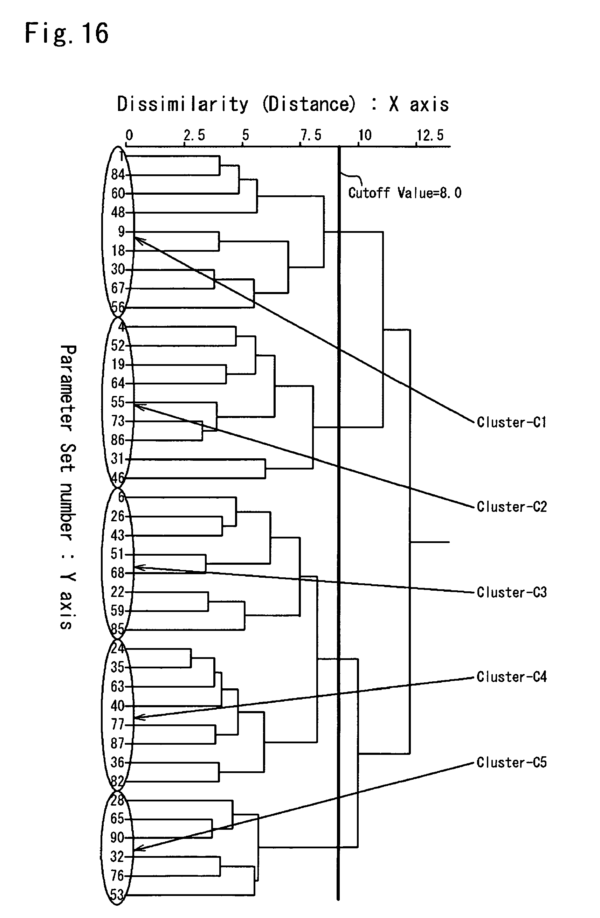

Subsequently, CPUA 110a executes the process of hierarchical cluster analysis on the parameter set PS candidates which have been standardized. FIG. 16 shows a dendrogram of cluster analysis result. Here, "Euclidian distance" is used for a reference value to decide whether individuals are similar or not and Ward's method is used to calculate distance.

When there are p pieces (p: 1 to 19) of parameters for n pieces (n: 1 to 100) of individuals (parameter set candidates), each parameter is expressed as $X_{i1}$, $X_{i2}$, ···, $X_{ip}$ (i: 1, 2, ···, n) . In an initial state, n-pieces of individuals each comprises a cluster and therefore n-pieces of clusters is considered to exist.

And a Euclidian square distance $d_{ij}{}^2$ between clusters (individuals in an initial state) is obtained by Formula 6.

[Formula 6]

$$d_{ij}^{2} = \sum_{k=1}^{p} \left( X_{ik} - X_{jk} \right)^{2} \quad (i, j, = 1, 2, \cdots, n) \quad \cdots\cdots\cdots(6)$$

When a Euclidian distance is obtained, clusters having the most approximate distance each other are consolidated to form a new cluster.

That means, when a new cluster c is formed by consolidation of cluster a and cluster b, a distance between clusters a and b before consolidation is set as $d_{ab}$, a distance between cluster a and other cluster x (x ≠ a, x ≠ b) is set as $d_{xa}$, and a distance between cluster b and other cluster x (x ≠ a, x ≠ b) is set as $d_{xb}$. A distance $d_{xc}$ between cluster c and other cluster x (x ≠ a, x ≠ b) is expressed by Formula . 7.

[Formula 7]

$$d_{xc}^{2} = \{(n_{x}+n_{a}) / (n_{x}+n_{c})\} d_{xa}^{2} + \{(n_{x}+n_{b}) / (n_{x}+n_{c})\} d_{xb}^{2}$$
$$+ \{-n_{x} / (n_{x}+n_{c})\} d_{ab}^{2} \quad \cdots\cdots\cdots(7)$$

Here, $n_a$ is the number of individuals included in cluster a (the number of parameter set candidates). The same is for $n_b$, $n_c$, $n_x$.

Consolidation of 2 clusters decrease the total number of clusters by 1 cluster. The cluster analysis is completed by repeating the consolidation process until the total cluster number is 1.

The calculated distance d shows a dissimilarity rate between individuals (parameter set candidates). The smaller distance, the more similar they are. FIG. 16 shows a part of dendrogram where X axis represents a dissimilarity rate (distance) between parameter set candidates and y axis represents parameter set candidates.

In FIG. 16, for example, a dissimilarity rate between parameter sets PS#01 and PS#84 is about 4. For example, when parameter set candidates having dissimilarity rate of less than 8 are collected to form a cluster, cutoff value = 8 is set and a group of parameter sets, each of which is located in a tip of branch cut off by the cutoff value (left side of cutoff in FIG. 16), forms a cluster. FIG. 16 shows 5 clusters C1 to C5 among 10 formed clusters in total.

Because values of parameter set candidates belonging to a generated cluster are approximate, functions are similar when they are applied to a biological model. Therefore, CPU 110a executes a process of generating a single parameter set candidate representing a cluster. To generate a single parameter set candidate (parameter set candidate representative for a cluster), for example, a mean value of parameters of each parameter set belonging to a cluster may be set as a parameter value of the parameter set candidate representative for the cluster. For obtaining a mean value of each parameter set, classification based on similarity (dissimilarity) may be weighed instead of single average.

A parameter set candidate of each cluster is obtained by the above processes, and therefore 100 sets of parameter candidates are to be narrowed down to 10 pieces of parameter set candidate whose parameter values are not approximate to each other.

[Step S3: generating a biological function profile of each cluster]

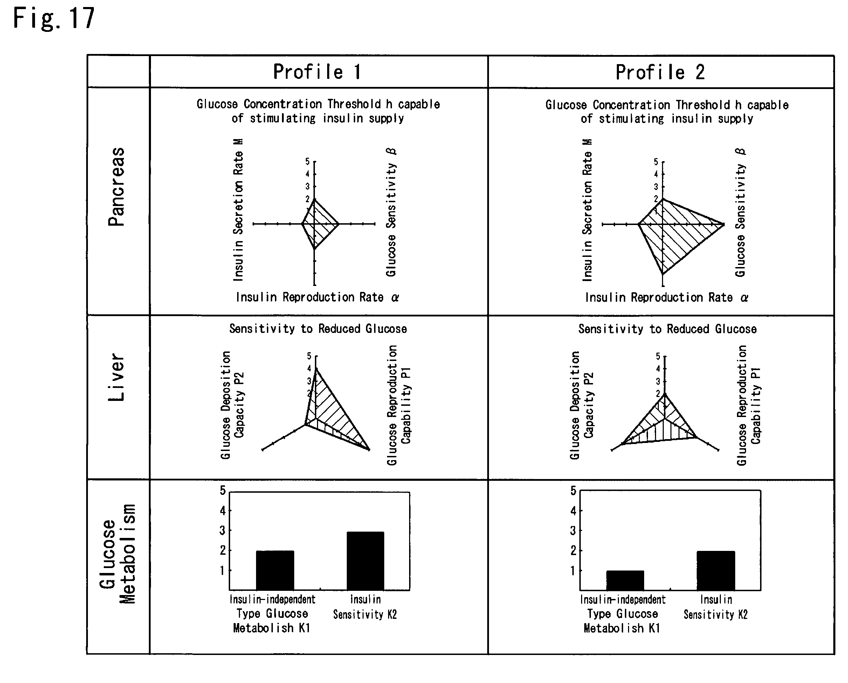

Further, this system generates a biological model function profile every 10 pieces of clusters based on each parameter value of parameter set candidate representative for a cluster (Step S-3). The biological function profile is generated for pancreas, liver, and glucose metabolism, for example, as shown in FIG. 17.

In a pancreas biological function profile, parameters of the pancreas model block, "glucose secretion rate (secretion rate per unit concentration) M", "threshold of glucose concentration capable of stimulating insulin supply h", "glucose sensitivity (sensitivity to glucose stimulation) β", and "insulin production rate (glucose stimulation following capability) α" are expressed in index of numeral values 1 to 5.

In a liver biological function profile, parameters of the hepatic metabolism model block, "glucose storage capacity (hepatic glucose uptake rate per unit insulin and unit glucose) P2", "glucose decrease sensitivity (glucose concentration basic value) Gb", and "glucose production capacity (glucose production rate for glucose stimulation lower than Gb) P1" are expressed in index of numeral values 1 to 5.

In a glucose metabolism biological function profile, parameters of the peripheral tissue model block, "insulin-independent glucose metabolism capacity (insulin-independent consumption rate in peripheral tissue) K1" and "insulin sensitivity (insulin-dependent glucose consumption rate in peripheral tissue) K2" are expressed in index of numeral values 1 to 5.

A reference value of each axis of biological function profiles is set as 3. A function exceeding the reference value is normal, and lower than the reference value shows higher degree of function failure. Two biological function profiles 1 and 2 in FIG. 17 are generated for two candidates among parameter set candidates representative for ten pieces of clusters mentioned above.

With regard to two biological function profiles for pancreas in FIG. 17, each item (each axis) of Profile 1 is less than a reference value of 3, which shows the pancreas is malfunctional. On the contrary, two items (glucose sensitivity and insulin production rate) in profile 2 is more than a standard value of 3, which shows pancreas is normal in terms of these items.

[Step S4: calculating physiological index]

Although biological model parameter set PS candidates to reproduce time-series data shown in FIG. 8 (a) (b) in a given error range are generated by GA procedure and grouped into a cluster as mentioned above, a plurality of candidates may be left in some cases. This is because those which do not emulate an actual biological organ are included even in the parameter set PS which can reproduce the time-series data of FIG. 8(a) (b). It means the number of biological model parameter sets capable of generating similar output is not single but plural that a plurality of candidates are left after grouping into a cluster. This shows there are some cases where parameter sets as an optical solution or a quasi-optical solution can not be narrowed down in Step S2 of estimating parameter set.

Therefore, this system has functions of narrowing down selecting acquiring parameter set candidates in different viewpoint (different reference) from that of Step S2. That means, the above-mentioned biological function profile is used for narrowing down and acquiring candidates here.

The biological function profile shows conditions of biological organs (pancreas) of biological model to which parameter set PS candidates are applied. This narrowing process is performed based on whether the conditions of biological function in the biological model shown by the profile are approximate to conditions shown by biological organ index which is obtained from actual living body's examination values (diagnosis data) including blood glucose level.

This calculation of the biological index (Step S4) is performed with CPU 100a based on the diagnosis data of the actual living body. In this embodiment, because OGTT time-series data (blood glucose level, insulin concentration; ref. to FIG. 8) are used as diagnosis data, another input processing from STEP S1 is not needed and therefore process is simple. However, types of diagnosis data are determined based on biological index calculated in Step S4 and when another input from that of Step 1 is needed, the diagnosis data are appropriately input.

In this embodiment, biological indexes used are HOMA-IR (insulin resistance index), HOMA-β (insulin secretion index), Insulinogenic index (insulin initial secretory capacity index); hereinafter referred to as "I index". These indexes can be calculated based on a blood glucose level and blood insulin concentration (input in Step S1). These indexes are calculated by CPU 110a based on information (blood glucose level, blood insulin concentration) handled in the biological model, where the value itself indicated by the index is the information which is not handled in the biological model. However, as a biological index, some values such as a parameter value may be correspondent to information handled in the biological model.

HOMA-IR is an index for glucose metabolism and HOMA-β and I index are indexes for pancreas conditions. As for physiological index, HbA1c (change rate of HbA1c) may be used.

Each index is obtained by the following formula.

$$\text{HOMA-IR} = (\text{fasting blood glucose level}) \times (\text{fasting insulin concentration}) / 405$$

$$HOMA - \beta = 360 \text{ X (fasting insulin concentration)/}$$
$$\text{(fasting blood glucose level - 63)}$$

$$\text{I index} = \text{(30 minute insulin concentration - fasting}$$
$$\text{insulin concentration) / (30 minute blood glucose level -}$$
$$\text{fasting blood glucose level)}$$

With HOMA-IR, less than 1 shows normal regarding insulin resistance and more than 2 shows resistant tendency and more than 3 shows obvious resistance.

With HOMA -β, normal level is 40 to 60 and the lower numerical value shows the lower secretory capacity.

With I index, more than 0.8 shows no problem, a range between 0.4 to 0.8 shows tendency to develop diabetes, less than 0.4 shows diabetes.

[Table 6]

| Blood Glucose Level Insulin Concentration with OGTT | | |
|---|---|---|
| Time | Blood Glucose Level | Insulin Concentration |
| 0 | 184 | 3 |
| 30 | 269 | 5 |
| 60 | 308 | 7 |
| 120 | 312 | 7 |
| 180 | 195 | 4 |

Table 6 shows numeral values of data in FIG. 8(a) (b) and Table 7 is a calculation result of each index based on table 6.

[Table 7]

| Physiological Index Calculation Result | | |
|---|---|---|
| Index | Value | Condition shown by Index |
| HOMA-β | 8.93 | Highly Secretory Failure |
| HOMA-IR | 1.36 | OK |
| I index | 0.02 | Diabetes |

[Step S5: Selecting biological function profile]

Subsequently, this system selects an appropriate biological function profile from the biological function profiles generated in Step S3 using the physiological indexes calculated in Step S5 (Step 5).

Particularly, approximation between a biological organ condition which is shown by a biological function profile and a biological condition which is shown by biological index is determined, an appropriate biological function profile is selected, and it is output to display 120 (Step 6). Through this process, approximation between the biological organ condition which is shown by parameter values included in an internal parameter set generated in Step S2 and the biological organ condition which is shown by physiological index obtained from an actual body examination are determined, so that an appropriate internal parameter set can be selected from a plurality of internal parameter sets generated.

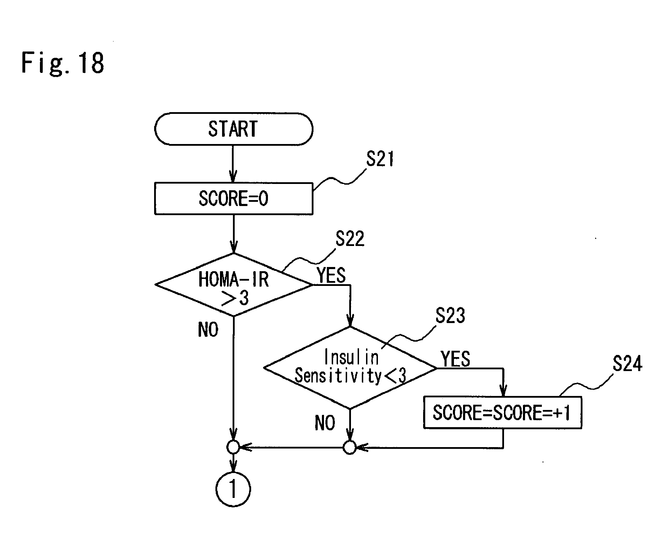

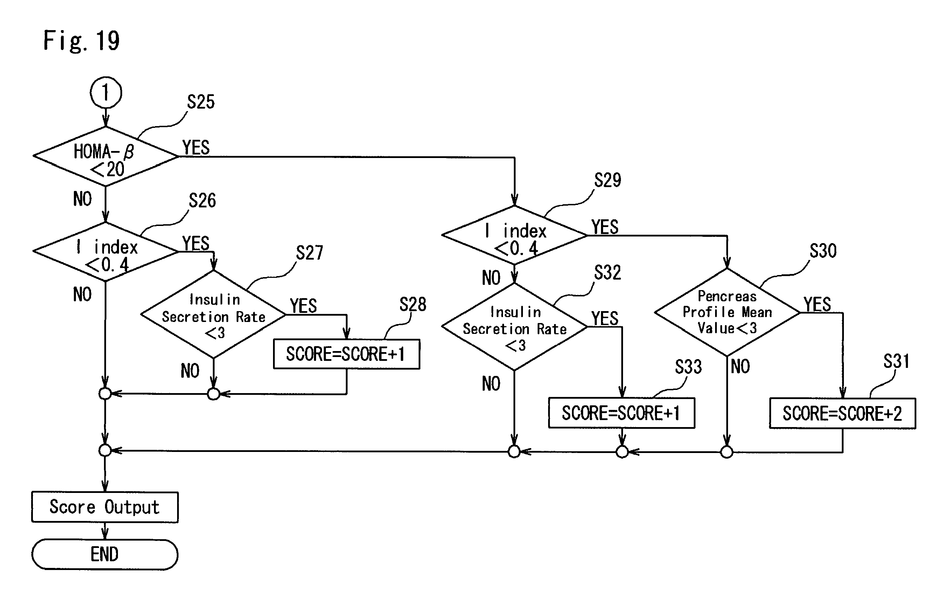

FIG. 18 and FIG. 19 show algorithm used for calculating scores as a reference value for selection. This algorithm is designed to increase scores and biological function profile with high scores are selected, when the biological function profile value and physiological index are abnormal and both are regarded as being approximate.

First, score = 0 is set as initial value (Step S21), and next, glucose metabolism condition or HOMA-IR are evaluated (Steps S22 to 24). In Step S22, determined is whether HOMA-IR i.e. insulin resistance index shows abnormal (HOMA-IR>3) or not. When it is determined as abnormal (YES), determined is whether sensitivity to insulin K2 of biological function profile is abnormal (K2<3) or not (Step S23). When the insulin sensitivity is also abnormal, score value is raised by 1 (Step S24). When either HOMA-IR or insulin sensitivity is not abnormal, score is not added.

Further, this system determines pancreas conditions, i.e. HOMA-β and I index (Steps S25 to S28, Steps S29 to S33). First, determined is whether HOMA-β, index of insulin secretory capacity shows great abnormality (HOMA-β<20) or not (Step S25). When I index shows abnormality (diabetes; index<0.4)(Step S26) although HOMA-β does not show great abnormality (NO), determined is whether insulin secretion rate M of biological function profile shows abnormality (M<3) or not (Step S27). When the insulin secretion rate shown by the profile is abnormal, a score value is added by 1 because the profile is matched with physiological index (Step S28). When the I index or insulin secretion rate M dose not show abnormality, the score value is not added.

When HOMA- β also shows great abnormality ("YES" in Step S25), determined is whether I index shows abnormality or not (Step S29) . When I index shows abnormality, abnormality degree is considered high. In this case, determined is whether mean value of all 4 items of pancreas profile is less than reference value of 3 or not (Step S30). When the mean value of pancreas profile is less than the reference value, the pancreas profile shows abnormality and the profile is highly matched with physiological index, therefore 2 is added to score value (Step S31) . However, the mean value of the pancreas profile exceeds the reference, the score value is not added.

When I index does not show abnormality ("NO" in Step S29), determined is whether insulin secretion rate M of the biological function profile shows abnormality (M < 3) or not (Step S32). When the profile shows abnormality of the insulin secretion rate, the profile is matched with the physiological index and 1 is added to score value (Step S33) . When the insulin secretion rate M does not show abnormality, the score value is not added.

For example, applied with the above mentioned score calculation algorithm to profile 1 and profile 2 shown in FIG. 16, the score becomes "2" "0" respectively. Therefore, it is known that profile 1 whose score is higher is appropriate.

Scores of biological function profile of each cluster is calculated based on the above-mentioned selection algorithm, one or more biological function profile having the highest score is selected as appropriate biological function profile (Step S5) and the profile is output to a display 120 (Step S6) . And then the parameter set candidate which is a basis on generation of appropriate biological function profile becomes a parameter set as an optimal solution or a quasi-optimal solution to be sought.

The present invention is not to be restricted to the above mentioned embodiments, and various modification may be possible without departing from the spirit and scope of the invention.

For example, a subject to be simulated is not limited to diabetes pathological conditions, but may be other pathological conditions. And constructions of biological model and its parameters are not limited to the above mentioned ones and may be changed accordingly.

Further, as a pathological index, other indexes including a liver index may be used. And in the above embodiment, parameter set candidates which are generated using genetic algorithm are narrowed down using biological function profile, but only parameter set generation by genetic algorithm may be conducted.

In addition, with regard to adaptability evaluation of the genetic algorithm in the embodiment, approximation between the biological model output and the actual biological response is used as evaluation reference. However, the evaluation reference may be approximation between conditions which are shown by the biological function profile and conditions which are shown by the biological index.

Further, in the above-mentioned embodiment, the parameter sets automatically generated are narrowed down with approximation between the biological model output and the actual body response, and then narrowed down with approximation between the conditions shown by the biological function profile and the conditions shown by the physiological index. However, the narrow-down process with approximation between the conditions shown by the biological function profile and the conditions shown by the physiological index may be conducted first, and then a narrow-down process with approximation between the biological model output and the actual body response may be conducted.

## Claims

1. A biological simulation system using a biological model comprising:

   an internal parameter set generating section generating internal parameter sets constituting a biological model; and
   a biological model computing section computing output of a biological model which emulates a biological response of a biological organ based on the generated internal parameter set,

wherein said internal parameter set generating section comprises:

a means for automatically generating a plurality of different internal parameter sets; and
a selecting means which determines an approximation between biological model output calculated applying the internal parameter set automatically generated and an actual biological response corresponding to said output and which selects an appropriate internal parameter set from a plurality of the generated internal parameter sets.

2. A biological simulation system using a biological model comprising:

an internal parameter set generating section generating internal parameter sets constituting a biological model; and
a biological model computing section simulating a biological organ based on the generated internal parameter set,
wherein said internal parameter set generating section comprises:

a means for automatically generating a plurality of different internal parameter sets; and
a selecting means which determines an approximation between a biological organ condition shown by a parameter value included in the generated internal parameter set and a biological organ condition shown by a physiological index obtained from an actual body examination and which selects an appropriate internal parameter set from a plurality of generated internal parameter sets.

3. A biological simulation system using a biological model comprising:

an internal parameter set generating section generating internal parameter sets constituting a biological model; and
a biological model computing section simulating a biological organ based on the generated internal parameter set,
wherein said internal parameter set generating section comprises:

a means for automatically generating a plurality of different internal parameter sets;
an obtaining means for obtaining internal parameter set showing a biological organ condition approximate to the biological organ condition shown by a biological index which is obtained from an actual body examination, based on the generated internal parameter set.

4. A biological simulation system using a biological model comprising:

an internal parameter set generating section generating internal parameter sets constituting a biological model; and
a biological model computing section computing output of the biological model which emulates a biological response of a biological organ based on the generated internal parameter set,
wherein said internal parameter set generating section comprises:

a means for automatically generating a plurality of different internal parameter sets;
a selecting means which determines an approximation between the biological model output calculated applying the internal parameter set automatically generated and an actual biological response corresponding to said output and which selects a plurality of internal parameter sets from a plurality of the generated internal parameter sets; and
an obtaining means for obtaining an appropriate internal parameter set showing a biological organ condition approximate to the biological organ condition shown by the biological index which is obtained from an actual body examination, based on a plurality of the internal parameter sets generated by said selecting means.

5. The biological simulation system according to claim 4, wherein said obtaining means comprises:

a cluster analyzing means which forms a cluster by grouping said internal parameter sets whose parameter values are similar to each other, and which generates an internal parameter set representative of said cluster; and
a selecting means for selecting appropriate internal parameter set showing a biological organ condition approximate to the biological organ condition shown by a biological index which is obtained from an actual body examination, from the internal parameter sets generated by said cluster analyzing means.

6. The biological simulation system according to any of claims 2 through 5, wherein said physiological index is information except one handled in a computing process of said biological model computing section.

7. The biological simulation system according to any of claims 2 through 6, wherein said physiological index includes at least one of HOMA-IR, HOMA-$\beta$, Insulinogenic index, and HbA1c.

8. A biological simulation system using a biological model comprising:

a biological model computing section computing output of a biological model which emulates a biological response of a biological organ based on an internal parameter set; and
an internal parameter set generating section generating internal parameter sets in which said output of the biological model approximate to an actual biological response when the internal parameter set is applied to the biological model,
wherein said internal parameter set generating section comprises:

a means for automatically generating a plurality of different internal parameter sets;
a selecting means which evaluates a plurality of the internal parameters automatically generated based on a first evaluation reference and narrows down a plurality of internal parameter sets from a plurality of said internal parameter sets based on the evaluation result; and
an obtaining means obtaining an internal parameter set matches a second evaluation reference which is different from said first evaluation reference, based on a plurality of the internal parameter sets selected by said selecting means.

9. The biological simulation system according to any of claims 1 through 8, wherein said biological model simulates physiological conditions of diabetes.

10. The biological simulation system according to any of claims 1 through 9, wherein said biological model receives a glucose intake amount as input and outputs a blood glucose level and a blood insulin concentration.

11. A computer program product stored in a computer-readable medium for processing a biological simulation which is executed by a computer, comprising:

a program code for generating internal parameter sets constituting a biological model; and
a program code for computing output of the biological model emulating a biological response of a biological organ based on the generated internal parameter set,
wherein said program code for generating said internal parameter sets comprises:

a program code for automatically generating a plurality of different internal parameter sets; and
a program code which determines an approximation between the output of the biological model which is calculated by applying automatically generated internal parameter sets and an actual biological response corresponding to said output, and which selects an appropriate internal parameter set from a plurality of the generated internal parameter sets.

Fig. 1

# Fig. 2

Fig. 3

Fig.4

Blood Glucose
Level BG

Insulin Secretion
Rate SR

Gb

P1

P2

K

Goff

Net glucose
Release Gout

Posthepatic
Insulin SRpost

# Fig. 5

Peripheral Tissue Insulin
Concentration $I_3$

Posthepatic Insulin
SRpost

Blood Insulin
Concentration $I_1$

# Fig. 6

Net Glucose Release from liver RGout

Glucose Absorption RG

Blood Glucose Level BG

Peripheral Tissue Insulin Concentration I3

51

52

53

54

55

56

57

1/S

K1

K2

...

# Fig. 7

```
┌─────────────────────────────┐
│            START            │
└─────────────────────────────┘
              │
              ▼
┌─────────────────────────────┐
│ Inputting OGTT Time-series  │ ── S1
│           Data              │
└─────────────────────────────┘
              │
              ▼
┌─────────────────────────────────────┐
│   Biological mathematical Model      │
│   Parameter Set Estimation Process   │ ── S2
│ ┌─────────────────────────────────┐  │
│ │ Generating Parameter Set        │  │ ── S2-1
│ │ Candidate by Stochastic         │  │
│ │ Optimization Method             │  │
│ └─────────────────────────────────┘  │
│ ┌─────────────────────────────────┐  │
│ │ Generating Cluster by           │  │ ── S2-2
│ │ Hierarchical Cluster Analysis   │  │
│ └─────────────────────────────────┘  │
└─────────────────────────────────────┘
              │
              ▼
┌─────────────────────────────┐
│ Generating Biological       │    S3
│ Function Profile per Cluster│
└─────────────────────────────┘
              │
              ▼
┌─────────────────────────────┐
│ Calculating Physiological   │ ── S4
│           Index             │
└─────────────────────────────┘
              │
              ▼
┌─────────────────────────────┐
│ Selecting Biological        │ ── S5
│ Function Profile            │
└─────────────────────────────┘
              │
              ▼
┌─────────────────────────────┐
│ Outputting Biological       │ ── S6
│ Function Profile            │
└─────────────────────────────┘
              │
              ▼
┌─────────────────────────────┐
│            END              │
└─────────────────────────────┘
```

# Fig. 8

(a)

**Blood Glucose Level (Actual measurement)**

Time t [min]

(b)

**Insulin Concentration (Actual measurement)**

Time t [min]

# Fig. 9

```
┌─────────────────────────────────┐
│      Genetic Algorithm          │
│   (Stochastic Optimization)     │
└─────────────────────────────────┘
                │
                ▼
┌─────────────────────────────────┐  S11
│     Generating Initial Group    │
└─────────────────────────────────┘
                │
                ▼
┌─────────────────────────────────┐  S12
│     Evaluating Fitness Rate     │
└─────────────────────────────────┘
                │
                ▼
       ◇─────────────────◇  S13
End ◁──  Determining End
       ◇─────────────────◇
                │ Not End
                ▼
┌─────────────────────────────────┐
│   ○  │ Selecting │ ～S14-1       │
│                                 │  S14
│   ○  │ Crossing  │ ～S14-2       │
│                                 │
│   ○  │ Mutating  │ ～S14-3       │
└─────────────────────────────────┘
                │
                ▼
       ◇─────────────────◇  S15
         Determining end     Not End
       ◇─────────────────◇
                │ End
                ▼
┌─────────────────────────────────┐
│              END                │
└─────────────────────────────────┘
```

Fig. 10

Reference (Actual Measured Blood Glucose Level)

Time t [min]

Fig. 11

Individual PS#01

Time t [min]

Fig. 12

Error between Individual PS#01 and Reference

Time t [min]

Fig. 13

| Parameter | PS#04 |
|---|---|
| P01 | 82.018 |
| P02 | 9.0354 |
| P03 | 103.76 |
| P04 | 333.56 |
| P05 | 0.019422 |
| P06 | 2493.3 |
| P07 | 76.125 |
| P08 | 0.00173 |
| P09 | 6.78E-06 |
| P10 | 5.0623 |
| P11 | 0.14734 |
| P12 | 0.66788 |
| P13 | 0.021914 |
| P14 | 0.14058 |
| P15 | 0.038294 |
| P16 | 0.066933 |
| P17 | 0.041225 |
| P18 | 0.024043 |
| P19 | 0.0004 |

| Parameter | PS#08 |
|---|---|
| P01 | 96.727 |
| P02 | 6.6845 |
| P03 | 70.688 |
| P04 | 278.29 |
| P05 | 0.015303 |
| P06 | 3418.9 |
| P07 | 108.84 |
| P08 | 0.002029 |
| P09 | 4.24E-06 |
| P10 | 4.5128 |
| P11 | 0.16826 |
| P12 | 0.37957 |
| P13 | 0.035786 |
| P14 | 0.13184 |
| P15 | 0.061049 |
| P16 | 0.046748 |
| P17 | 0.028912 |
| P18 | 0.017696 |
| P19 | 0.000854 |

| Parameter | New PS#01 |
|---|---|
| P01 | 82.018 |
| P02 | 9.0354 |
| P03 | 103.76 |
| P04 | 333.56 |
| P05 | 0.015303 |
| P06 | 2493.3 |
| P07 | 76.125 |
| P08 | 0.00173 |
| P09 | 6.78E-06 |
| P10 | 5.0623 |
| P11 | 0.16826 |
| P12 | 0.66788 |
| P13 | 0.021914 |
| P14 | 0.14058 |
| P15 | 0.038294 |
| P16 | 0.066933 |
| P17 | 0.041225 |
| P18 | 0.024043 |
| P19 | 0.0004 |

| Parameter | New PS#02 |
|---|---|
| P01 | 96.727 |
| P02 | 6.6845 |
| P03 | 70.688 |
| P04 | 278.29 |
| P05 | 0.019422 |
| P06 | 3418.9 |
| P07 | 108.84 |
| P08 | 0.002029 |
| P09 | 4.24E-06 |
| P10 | 4.5128 |
| P11 | 0.14734 |
| P12 | 0.37957 |
| P13 | 0.035786 |
| P14 | 0.13184 |
| P15 | 0.061049 |
| P16 | 0.046748 |
| P17 | 0.028912 |
| P18 | 0.017696 |
| P19 | 0.000854 |

# Fig. 14

| Parameter | PS#01 | PS#02 | PS#03 | PS#04 |
|---|---|---|---|---|
| P01 | 77.821 | 82.033 | 94.606 | 82.379 |
| P02 | 6.5828 | 9.8641 | 9.8654 | 7.0707 |
| P03 | 55.022 | 129.14 | 92.681 | 48.874 |
| P04 | 322.68 | 158.78 | 148.37 | 283.16 |
| P05 | 0.0199 | 0.01913 | 0.019994 | 0.017849 |
| P06 | 3667 | 4402.4 | 4376.9 | 4120.8 |
| P07 | 128.84 | 96.993 | 135.67 | 79.909 |
| P08 | 0.001177 | 0.000954 | 0.000701 | 0.001951 |
| P09 | 2.1E-06 | 2.93E-06 | 5.08E-06 | 7.63E-06 |
| P10 | 3.9611 | 3.3664 | 3.9905 | 5.6773 |
| P11 | 0.19158 | 0.18024 | 0.1823 | 0.16922 |
| P12 | 0.69099 | 0.68778 | 0.68144 | 0.48112 |
| P13 | 0.024476 | 0.035714 | 0.035171 | 0.043875 |
| P14 | 0.29821 | 0.23586 | 0.30913 | 0.20052 |
| P15 | 0.049789 | 0.060879 | 0.060842 | 0.057303 |
| P16 | 0.038031 | 0.041882 | 0.068396 | 0.085411 |
| P17 | 0.034139 | 0.064749 | 0.056596 | 0.031404 |
| P18 | 0.020108 | 0.017729 | 0.017046 | 0.03625 |
| P19 | 0.000757 | 0.000657 | 0.000533 | 0.000413 |

$\sim$

| PS#97 | PS#98 | PS#99 | PS#100 |
|---|---|---|---|
| 81.848 | 88.973 | 82.599 | 89.972 |
| 8.5265 | 7.7775 | 10.056 | 9.4005 |
| 47.889 | 107.82 | 69.734 | 127.62 |
| 137.98 | 319.84 | 155.06 | 245.02 |
| 0.019983 | 0.017048 | 0.018749 | 0.019162 |
| 3444.9 | 4005.6 | 2395.5 | 4350.4 |
| 65.597 | 56.904 | 151.51 | 61.341 |
| 0.00205 | 0.001402 | 0.000787 | 0.001787 |
| 2.28E-06 | 2.62E-06 | 5.4E-06 | 4.73E-06 |
| 4.3355 | 7.0191 | 4.9057 | 7.1136 |
| 0.15079 | 0.16955 | 0.18457 | 0.14973 |
| 0.74786 | 0.47006 | 0.58862 | 0.59188 |
| 0.040283 | 0.043936 | 0.024556 | 0.023531 |
| 0.27701 | 0.18187 | 0.11489 | 0.19855 |
| 0.047233 | 0.051684 | 0.036193 | 0.036957 |
| 0.075596 | 0.053133 | 0.33324 | 0.03901 |
| 0.025664 | 0.067064 | 0.04952 | 0.082559 |
| 0.017533 | 0.042791 | 0.033157 | 0.039697 |
| 0.000434 | 0.000769 | 0.000608 | 0.000843 |

EP 1 722 344 A1

## Fig. 15

| Parameter | nPS#01 | nPS#02 | nPS#03 | nPS#04 |
|---|---|---|---|---|
| P01 | 1.604557 | 1.691402 | 1.950639 | 1.698536 |
| P02 | 1.2807 | 1.919086 | 1.919339 | 1.375623 |
| P03 | 0.782674 | 1.836984 | 1.318364 | 0.69522 |
| P04 | 1.926448 | 0.94794 | 1.885791 | 1.690507 |
| P05 | 1.99 | 1.913 | 1.9994 | 1.7549 |
| P06 | 1.594348 | 1.914087 | 1.903 | 1.791652 |
| P07 | 1.6105 | 1.212413 | 1.695875 | 0.995563 |
| P08 | 1.014397 | 0.822129 | 0.604517 | 1.681552 |
| P09 | 0.50387 | 0.704591 | 1.220216 | 1.835168 |
| P10 | 1.042395 | 0.885895 | 1.050132 | 1.492974 |
| P11 | 1.987344 | 1.86971 | 1.891079 | 1.755394 |
| P12 | 1.577603 | 1.570274 | 1.555799 | 1.098447 |
| P13 | 1.112545 | 1.623364 | 1.598682 | 1.994318 |
| P14 | 1.537165 | 1.215773 | 1.593454 | 1.033608 |
| P15 | 0.743119 | 0.908642 | 0.90809 | 0.855269 |
| P16 | 0.731365 | 0.805423 | 1.315308 | 1.642519 |
| P17 | 0.812833 | 1.541643 | 1.347524 | 0.747714 |
| P18 | 0.591412 | 0.521441 | 0.501353 | 1.066176 |
| P19 | 1.610149 | 1.398128 | 1.133596 | 0.879489 |

~

| nPS#97 | nPS#98 | nPS#99 | nPS#100 |
|---|---|---|---|
| 1.677278 | 1.687588 | 1.834495 | 1.703072 |
| 1.215739 | 1.658852 | 1.513132 | 1.95642 |
| 1.635562 | 0.681209 | 1.533713 | 0.991949 |
| 1.03003 | 0.823761 | 1.909493 | 0.925731 |
| 1.9285 | 1.9983 | 1.7048 | 1.8749 |
| 1.274174 | 1.497783 | 1.741565 | 1.041522 |
| 1.72575 | 0.819963 | 0.7113 | 1.893875 |
| 0.983793 | 1.767414 | 1.208621 | 0.678353 |
| 0.958678 | 0.519135 | 0.629375 | 1.29863 |
| 1.568158 | 1.140921 | 1.847132 | 1.302816 |
| 1.885166 | 1.564212 | 1.758817 | 1.914627 |
| 1.353904 | 1.707443 | 1.073196 | 1.343881 |
| 1.8015 | 1.831045 | 1.997091 | 1.116182 |
| 1.17799 | 1.427887 | 0.937474 | 0.592216 |
| 0.509776 | 0.70497 | 0.771403 | 0.540194 |
| 0.751558 | 1.453769 | 1.02788 | 0.640846 |
| 0.746095 | 0.611048 | 1.596762 | 1.179048 |
| 0.888559 | 0.515676 | 1.258559 | 0.975206 |
| 1.659191 | 0.922617 | 1.63666 | 1.293532 |

EP 1 722 344 A1

# Fig. 16

Dissimilarity (Distance) : X axis

Parameter Set number : Y axis

Cutoff Value=8.0

Cluster-C1

Cluster-C2

Cluster-C3

Cluster-C4

Cluster-C5

Fig. 17

| | Profile 1 | Profile 2 |
|---|---|---|
| Pancreas | Glucose Concentration Threshold h capable of stimulating insulin supply; Glucose Sensitivity β; Insulin Reproduction Rate α; Insulin Secretion Rate M | Glucose Concentration Threshold h capable of stimulating insulin supply; Glucose Sensitivity β; Insulin Reproduction Rate α; Insulin Secretion Rate M |
| Liver | Sensitivity to Reduced Glucose; Glucose Reproduction Capability P1; Glucose Deposition Capacity P2 | Sensitivity to Reduced Glucose; Glucose Reproduction Capability P1; Glucose Deposition Capacity P2 |
| Glucose Metabolism | Insulin-independent Type Glucose Metabolish K1; Insulin Sensitivity K2 | Insulin-independent Type Glucose Metabolish K1; Insulin Sensitivity K2 |

EP 1 722 344 A1

Fig. 18

```
        ┌──────────────┐
        │    START     │
        └──────┬───────┘
               │
               ▼                    S21
        ┌──────────────┐
        │   SCORE=0    │
        └──────┬───────┘
               │
               ▼              S22
              ╱ ╲        YES
             ╱HOMA-IR╲──────────┐
             ╲  >3   ╱          │
              ╲ ╱               ▼           S23
               │ NO            ╱ ╲      YES
               │              ╱Insulin ╲──────────┐
               │              ╲Sensitivity<3      │
               │               ╲ ╱                ▼        S24
               │                │ NO      ┌──────────────────┐
               │                │         │  SCORE=SCORE=+1  │
               │                │         └────────┬─────────┘
               ▼                ▼                  │
               ○◄───────────────○◄─────────────────┘
               │
               ▼
              (1)
```

Fig. 19

European Patent
Office

**EUROPEAN SEARCH REPORT**

Application Number

EP 06 00 9775

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/071141 A1 (BUTLER RICHARD) 31 March 2005 (2005-03-31) * title * * paragraph [0092] * ----- | 1 | INV. G09B23/28 G06F17/50 G06F19/00 |
| X,P | EP 1 580 682 A (SYSMEX CORPORATION) 28 September 2005 (2005-09-28) * the whole document * ----- | 1 | |
| A | EP 1 418 523 A (SYSMEX CORPORATION) 12 May 2004 (2004-05-12) * the whole document * * paragraph [0094] * ----- | 1,7,9,10 | |
| A | US 2003/058245 A1 (BRAZHNIK PAUL ET AL) 27 March 2003 (2003-03-27) * the whole document * * paragraph [0140] * ----- | 1,7 | |
| A | LEHMANN E D ET AL: "Computer assisted diabetes care: A 6-year retrospective" COMPUT METHODS PROG BIOMED; COMPUTER METHODS AND PROGRAMS IN BIOMEDICINE AUG 1996 ELSEVIER SCIENCE IRELAND LTD, SHANNON, IRELAND, vol. 50, no. 3, August 1996 (1996-08), pages 209-230, XP002395785 * the whole document * * figure 4 * ----- -/-- | 1 | TECHNICAL FIELDS SEARCHED (IPC) G06F G09B |

The present search report has been drawn up for all claims

| Place of search | Date of completion of the search | Examiner |
|---|---|---|
| Munich | 23 August 2006 | Wellisch, J.P. |

EPO FORM 1503 03.82 (P04C01)

**European Patent Office**

**EUROPEAN SEARCH REPORT**

Application Number

EP 06 00 9775

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PILLONETTO GIANLUIGI ET AL: "Minimal model SI=0 problem in NIDDM subjects: Nonzero Bayesian estimates with credible confidence intervals" AMERICAN JOURNAL OF PHYSIOLOGY, vol. 282, no. 3 Part 1, March 2002 (2002-03), pages E564-E573, XP002395786 ISSN: 0002-9513 * the whole document * ----- | 1 | |
| | | | TECHNICAL FIELDS SEARCHED (IPC) |

The present search report has been drawn up for all claims

| Place of search | Date of completion of the search | Examiner |
|---|---|---|
| Munich | 23 August 2006 | Wellisch, J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 00 9775

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2006

| Patent document cited in search report | | Publication date | Patent family member(s) | | Publication date |
|---|---|---|---|---|---|
| US 2005071141 | A1 | 31-03-2005 | CA | 2459673 A1 | 20-03-2003 |
| | | | EP | 1428165 A2 | 16-06-2004 |
| | | | WO | 03023682 A2 | 20-03-2003 |
| | | | GB | 2381907 A | 14-05-2003 |
| | | | JP | 2005502406 T | 27-01-2005 |
| EP 1580682 | A | 28-09-2005 | CN | 1670755 A | 21-09-2005 |
| | | | JP | 2005267042 A | 29-09-2005 |
| | | | US | 2005234311 A1 | 20-10-2005 |
| EP 1418523 | A | 12-05-2004 | JP | 2004154341 A | 03-06-2004 |
| | | | US | 2004091424 A1 | 13-05-2004 |
| US 2003058245 | A1 | 27-03-2003 | CA | 2445598 A1 | 07-11-2002 |
| | | | EP | 1389998 A2 | 25-02-2004 |
| | | | JP | 2005508025 T | 24-03-2005 |
| | | | WO | 02087506 A2 | 07-11-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERGMAN'S.** *American Journal of Physiology,* 1979, vol. 236-6, E-667-77

- **BERGMAN et al.** *Journal of Clinical Investigation,* 1981, vol. 68 (6), 1456-67